# FASCICULE DE BREVET EUROPEEN

(11) **EP 1 086 684 B1**
(45) Date de publication et mention de la délivrance du brevet: **14.04.2010**
(21) Numéro de dépôt: 00402544.1
(22) Date de dépôt: 14.09.2000
(51) Int. Cl.: A61K 8/58, A61K 8/66, A61Q 5/10

(54) **Composition de teinture d'oxydation des fibres kératiniques et procédé de teinture mettant en oeuvre cette composition**
Oxidationsfärbemittel für keratinische Fasern und Färbungsverfahren mit diesem Mittel
Composition for oxidative dyeing of keratinous fibres and dyeing process using the same

(30) Priorité: 24.09.1999 FR 9911967
(43) Date de publication de la demande: 28.03.2001
(73) Titulaire: L'Oréal, 75008 Paris (FR)
(72) Inventeur: Plos, Grégory, 75015 Paris (FR); Lagrange, Alain, 77700 Coupvray (FR)
(74) Mandataire: Miszputen, Laurent

(56) Documents cités:
- EP-A- 0 310 675
- EP-A- 0 504 005
- EP-A- 0 548 620
- WO-A-98/44906
- D. AVNIR: "Enzymes and Other Proteins Entrapped in Sol-Gel Materials" CHEM. MATER., vol. 6, 1994, pages 1605-1614, XP000938822
- DATABASE CHEMABS [en ligne] CHEMICAL ABSTRACTS SERVICE, COLUMBUS, OHIO, US; INABA, RYUICHI ET AL: "Sol-gel preparation of organic colorant-doped silica gel and its properties" retrieved from STN Database accession no. 123:296224 CA XP002145934 & J. SCCJ (1995), 29(2), 154-62, 1995,

## Description

L'invention a pour objet une composition prête à l'emploi pour la teinture d'oxydation des fibres kératiniques, et en particulier des fibres kératiniques humaines telles que les cheveux comprenant, dans un milieu approprié pour la teinture, au moins un colorant d'oxydation, et au moins un système enzymatique dont l'enzyme de type oxydo-réductase à 2 ou à 4 électrons ou peroxydase est immobilisée dans une matrice de matériau obtenu par voie sol-gel, ainsi que le procédé de teinture mettant en oeuvre cette composition.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des bases hétérocycliques, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les méta-aminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

La coloration d'oxydation des fibres kératiniques est généralement réalisée en milieu alcalin, en présence de peroxyde d'hydrogène. Toutefois, l'utilisation des milieux alcalins en présence de quantités notables de peroxyde d'hydrogène présentent pour inconvénient d'entraîner une dégradation non négligeable des fibres, ainsi qu'une décoloration importante des fibres kératiniques qui n'est pas toujours souhaitable.

La coloration d'oxydation des fibres kératiniques peut également être réalisée à l'aide de systèmes oxydants différant du peroxyde d'hydrogène tels que des systèmes enzymatiques. Ainsi il a déjà été proposé de teindre les fibres kératiniques, notamment dans la demande de brevet EP-A-0 310 675, avec des compositions comprenant une base d'oxydation et éventuellement un coupleur, en association avec des enzymes du type oxydo-réductases à 2 électrons, telles que la pyranose-oxydase, la glucose-oxydase ou bien l'uricase, en présence d'un donneur pour lesdites enzymes.
Il a déjà également été proposé, notamment dans les demandes de brevets FR-A-2112549, FR-A-2694018, EP-A-504005, WO 95/07988, W095/33836, W095/33837, W096/00290, W097/19998, WO97/19999 et brevet US-3251742, de teindre les fibres kératiniques avec des compositions comprenant notamment un précurseur de colorant d'oxydation et une enzyme de type laccase (oxydo-réductase à 4 électrons).
Enfin, on a également proposé d'utiliser, pour teindre les fibres kératiniques, de la peroxydase en présence de faibles quantités de peroxyde d'hydrogène, comme on l'a décrit dans les brevets EP-548620, BE-775110 et US-3893803.
Ces procédés de teinture, bien qu'étant mis en oeuvre dans des conditions n'entraînant pas une dégradation des fibres kératiniques comparable à celle engendrée par les teintures réalisées en présence de quantités notables de peroxyde d'hydrogène, conduisent à des colorations ne donnant pas encore entière satisfaction.
En effet, des interactions existent entre, d'une part, le colorant ou les différents constituants du support de teinture et notamment les solvants et les épaississants, et d'autre part, lesdites enzymes. Ces interactions ont pour effet de diminuer l'efficacité desdites enzymes en teinture capillaire.
Par ailleurs, l'insuffisance de stabilité thermique de ce type d'enzyme dans les formulations de teinture, représente un autre inconvénient pour la teinture.

Or, la demanderesse vient maintenant de découvrir qu'il est possible d'obtenir de nouvelles teintures puissantes ne présentant plus ces inconvénients, en associant au colorant d'oxydation, un système enzymatique dont l'enzyme de type oxydo-réductase à 2 électrons ou à 4 électrons ou peroxydase est immobilisée dans une matrice de matériau obtenu par voie sol-gel.

Cette découverte est à la base de la présente invention.

Grâce à la présente invention, il est devenu possible, de diminuer la quantité d'enzyme utilisée, de diminuer voire empêcher les interactions entre le colorant ou les constituants du support de la composition de teinture avec l'enzyme, et d'améliorer en outre la stabilité thermique des compositions de teinture à base de ce type d'enzyme.

L'invention a donc pour premier objet, une composition prête à l'emploi pour la teinture d'oxydation des fibres kératiniques et en particulier des fibres kératiniques humaines telles que les cheveux, comprenant dans un milieu approprié pour la teinture, au moins un colorant d'oxydation et au moins un système enzymatique choisi parmi :
(a) au moins une oxydoréductase à 2 électrons associée à un donneur,
(b) au moins une oxydoréductase à 4 électrons,
(c)
   (i) au moins une peroxydase éventuellement associée à un donneur et
   (ii) du peroxyde d'hydrogène, ou un système enzymatique le générant in situ,
caractérisée par le fait que ladite enzyme est immobilisée dans une matrice de matériau obtenu par hydrolysé acide, ou basique partielle ou totale de précurseurs organométalliques, puis polycondensation, les précurseurs organométalliques sont des oxydes métalliques choisis dans le groupe formé par :
(1) les silanes, les titanates ou zirconates répondant à l'une des formules suivantes :

   M(OR₁)₄ (Ia)

   R-M(OR₁)₃ (Ib)

   ₃(OR₁) M (OR₁)₃ (Ic)

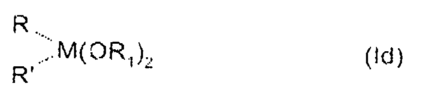

   dans lesquelles :
   M désigne Si, Ti ou Zr ;
   R₁ désigne un radical alkyle linéaire ou ramifié, de préférence en C₁-C₄ ;
   R et R' indépendamment l'un de l'autre, désignent un radical alkyle C₁-C₃₀ linéaire ou ramifie, un radical cycloalkyle C₁-C₃₀. un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy.

Par composition prête à l'emploi, on entend toute composition destinée à être appliquée immédiatement sur les fibres kératiniques en présence d'air.

L'invention a également pour objet un procédé de teinture d'oxydation des fibres kératiniques mettant en oeuvre cette composition tinctoriale prête à l'emploi.

La ou les oxydo-réductases à 2 électrons sont utilisées dans la composition tinctoriale prête à l'emploi conforme à l'invention, en présence d'un donneur pour la ou lesdites enzymes; elles peuvent notamment être choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

Selon l'invention, on entend par donneur, les différents substrats également nécessaires au fonctionnement de ladite ou desdites enzymes.

Selon l'invention, l'oxydo-réductase à 2 électrons est de préférence choisie parmi les uricases d'origine animale, microbiologique ou biotechnologique.

A titre d'exemple, on peut notamment citer l'uricase extraite de foie de sanglier, l'uricase d'Arthrobacter globiformis, ainsi que l'uricase d'Aspergillus flavus.

La ou les oxydo-réductases à 2 électrons peuvent être utilisées sous forme cristalline pure ou sous une forme diluée dans un diluant inerte pour ladite oxydo-réductase à 2 électrons.

La ou les oxydo-réductases à 2 électrons conformes à l'invention représentent de préférence de 0,01 à 20 % en poids environ du poids total de la composition, et encore plus préférentiellement de 0,1 à 10 % en poids environ de ce poids. On peut aussi définir la quantité d'enzyme en fonction de son activité.

L'activité enzymatique des oxydoréductases à 2 électrons conformes à l'invention peut être définie à partir de l'oxydation du donneur en condition aérobie.
Une unité U correspond à la quantité d'enzyme conduisant à la génération d'une µmole de H₂O₂ par minute à un pH de 8,5 et à une température de 25°C. De façon préférentielle, la quantité d'oxydoréductase à 2 électrons conforme à l'invention est comprise entre 10 et 10⁸ unités U environ pour 100g de composition tinctoriale.

La nature du donneur (ou substrat) pour ladite enzyme varie en fonction de la nature de l'oxydo-réductase à 2 électrons qui est utilisée.
Par exemple, à titre de donneur pour les pyranose oxydases, on peut citer le D-glucose, le L-sorbose et le D-xylose ; à titre de donneur pour les glucose oxydases, on peut citer le D-glucose; à titre de donneur pour les glycérol oxydases, on peut citer le glycérol et la dihydroxyacétone ; à titre de donneur pour les lactate oxydases, on peut citer l'acide lactique et ses sels ; à titre de donneur pour les pyruvate oxydases, on peut citer l'acide pyruvique et ses sels ; à titre de donneur pour les uricases, on peut citer l'acide urique et ses sels ; à titre de donneur pour les choline oxydases, on peut citer la choline et ses sels d'addition avec un acide comme le chlorhydrate de choline, et la bétaïne aldéhyde ; à titre de donneur pour les sarcosine oxydases, on peut citer la sarcosine, la N-méthyl-L-leucine, la N-méthyl-DL-alanine, et la N-méthyl-DL-valine ; à titre de donneur pour les bilirubine oxydases, on peut citer la bilirubine ; et enfin, pour les aminoacides oxydases, on peut citer pour les L-amino-acides oxydases, la L-glycine, le L-alanine, la L-valine, la L-phénylalanine et le L-tryptophane et pour les D-amino-acides oxydases, la D-alanine, et la D-phénylalanine.

Le ou les donneurs (ou substrats) utilisés conformément à l'invention représentent de préférence de 0,01 à 20 % en poids environ du poids total de la composition conforme à l'invention et encore plus préférentiellement de 0,1 à 10 % en environ de ce poids.

La ou les oxydo-réductases à 4 électrons utilisées dans la composition tinctoriale prête à l'emploi conforme à l'invention peuvent notamment être choisies parmi les laccases, les tyrosinases, les catéchol oxydases, les déaminooxydases et les polyphénols oxydases.

Selon une forme de réalisation particulière et préférée de l'invention la ou les oxydo-réductases à 4 électrons sont choisies parmi les laccases.

Ces laccases peuvent notamment être choisies parmi les laccases d'origine végétale, d'origine animale, d'origine fongique (levures, moisissures, champignons) ou d'origine bactérienne, les organismes d'origine pouvant être mono- ou pluricellulaires. Les laccases peuvent également être obtenues par biotechnologie.

Parmi les laccases d'origine végétale utilisables selon l'invention, on peut citer les laccases produites par des végétaux effectuant la synthèse chlorophyllienne telles que celles indiquées dans la demande de brevet FR-A-2 694 018.
On peut notamment citer les laccases présentes dans les extraits d'Anacardiacées tels que par exemple les extraits de Magnifera indica, de Schinus molle ou de Pleiogynium timoriense ; dans les extraits de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

Parmi les laccases d'origine fongique, éventuellement obtenues par biotechnologie, utilisables selon l'invention, on peut citer la ou les laccases issues de Polyporus versicolor, de Rhizoctonia praticola et de Rhus vernicifera telles que décrites par exemples dans les demandes de brevet FR-A-2 112 549 et EP-A-504005 ; les laccases décrites dans les demandes de brevet WO95/07988, WO95/33836, WO95/33837, WO96/00290, WO97/19998 et WO97/19999, dont le contenu fait partie intégrante de la présente description comme par exemple la ou les laccases issues de Scytalidium, de Polyporus pinsitus, de Myceliophtora thermophila, de Rhizoctonia solani, de Pyricularia orizae, et leurs variantes. On peut également citer la ou les laccases issues de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

On choisira plus préférentiellement les laccases d'origine fongiques, éventuellement obtenues par biotechnologie.
L'activité enzymatique des laccases utilisées conformément à l'invention et ayant la syringaldazine parmi leurs substrats peut être définie à partir de l'oxydation de la syringaldazine en condition aérobie. L'unité Lacu correspond à la quantité d'enzyme catalysant la conversion de 1 mmole de syringaldazine par minute à un pH de 5,5 et à une température de 30°C. L'unité U correspond à la quantité d'enzyme produisant un delta d'absorbance de 0,001 par minute, à une longueur d'onde de 530 nm, en utilisant la syringaldazine comme substrat, à 30°C et à un pH de 6,5. L'activité enzymatique des laccases utilisées selon l'invention peut aussi être définie à partir de l'oxydation de la paraphénylènediamine. L'unité ulac correspond à la quantité d'enzyme produisant un delta d'absorbance de 0,001 par minute, à une longueur d'onde de 496,5 nm, en utilisant la paraphénylènediamine comme substrat (64 mM), à 30°C et à un pH de 5.

De manière générale, la ou les oxydo-réductases à 4 électrons conformes à l'invention représentent de préférence de 0,01 à 20 % en poids environ du poids total de la composition tinctoriale prête à l'emploi, et encore plus préférentiellement de 0,1 à 10% en poids environ de ce poids.

De manière particulière, et lorsqu'une ou plusieurs laccases sont utilisées, la quantité de laccase(s) présente dans la composition tinctoriale prête à l'emploi conforme à l'invention variera en fonction de la nature de la ou des laccases utilisées. De façon préférentielle, la quantité de laccase(s) est comprise entre 0,5 et 2000 Lacu environ (soit entre 10000 et 40.10⁶ unités U environ ou soit entre 20 et 20.10⁶ unités ulac) pour 100 g de composition tinctoriale prête à l'emploi.

La ou les peroxydases utilisées dans la composition tinctoriale prête à l'emploi conforme à l'invention, peuvent notamment être choisies parmi les enzymes appartenant à la sous-classe 1.11.1 décrite dans l'ouvrage Enzyme Nomenclature, Academic Press Inc., 1984. Certaines d'entre elles nécessitent la présence d'un donneur pour fonctionner. C'est le cas, en particulier des NADH peroxydases (1.11.1) [donneur = NADH], des acides gras peroxydases (1.11.1.3) [donneur = acide gras, par exemple palmitate] , des NADPH peroxydases (1.11.1.2), [donneur = NADPH], des cytochrome-c peroxydases (1.11.1.5) [donneur = ferrocytochrome c], des iodures peroxydases (1.11.1.8) [donneur = iodure], des chlorures peroxydases (1.11.10) [donneur = chlorure], des L. ascorbates peroxydases (1.11.1.11) [donneur = Lascorbate], des glutathion peroxydases (1.11.1.9) [donneur = glutathion].
D'autres peroxydases fonctionnent sans donneur autre que le colorant d'oxydation ; il en est ainsi des catalases (1.11.1.6) et des peroxydases simplex (1.11.1.7).

Selon l'invention, on utilise de préférence les peroxydases simplex (1.11.1.7).

Toutes les peroxydases fonctionnent en présence de peroxyde d'hydrogène qui est apporté tel quel ou généré in situ par voie enzymatique [oxydase(s) à 2 électrons et donneur(s) en présence d'air].

Les peroxydases utilisées peuvent être d'origine végétale, animale, fongique, bactérienne. Elles peuvent être également obtenues par biotechnologie.
Ainsi, les peroxydases peuvent par exemple être issues de la pomme, de l'abricot, de l'orge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acetobacter peroxidans, Staphylococcus faecalis, Arthromycesramosus.

L'unité d'activité de la peroxydase simplex (1.11.1.7) peut se définir comme étant la quantité d'enzyme simplex formant 1mg de purpurogalline à partir du pyrogallol en 20s à pH6 et à 20°C. A titre d'exemple, la peroxydase de radis noir P6782 de SIGMA présente une activité d'environ 250 unités par mg.
La concentration d'utilisation de ce type d'enzyme varie donc entre 25 et 5.10⁶ unités pour 100g de composition.

La ou les peroxydases conformes à l'invention représentent de préférence de 0,0001 à 20% en poids environ du poids total de la composition, et encore plus préférentiellement de 0,001 à 10% en poids environ de ce poids.

Le procédé sol-gel est bien connu de l'art antérieur, voir à ce sujet l'article de C.J. Brinker et G.W. Scherer dans Sol-Gel Science - Academic Press : New York, 1990 ; c'est une technique de synthèse chimique qui conduit à la préparation de gels et de verres transparents à partir d'alcoxydes métalliques , lesquels engendrent lesdits gels à température ambiante par hydrolyse partielle ou totale et polymérisation par condensation.
Le procédé sol-gel est connu pour immobiliser des biomolécules et notamment des enzymes, voir à ce sujet B. Dunn et J.M. Miller dans la revue Acta Mater. Vol 46 - N°3 - pp737-741- 1998, ou Lisa M. Ellerby dans Science -28 février 1992-Vol 255 - pp1041-1180 ou encore D. Avnir dans Chem. Mater. 1994 - 6 - pp1605-1614.

La réaction sol-gel est faite avec des précurseurs organométalliques en présence d'une quantité suffisante d'eau ajoutée et/ou provenant de l'humidité ambiante et éventuellement en présence d'un ou plusieurs solvants organiques cosmétiquement acceptables.

Les solvants organiques utilisés peuvent être choisis par exemple parmi :
- les alcools inférieurs en C₁-C₄ tels que l'éthanol ;
- le propylène glycol, les esters et les éthers de propylène glycol ;
- l'éthylène glycol, les esters et les éthers de l'éthylène glycol ;
- l'acétone ou la méthyléthylcétone ;
- l'acétate de méthyle, d'éthyle ou de butyle ;
- le glycérol ;
- les huiles carbonées volatiles telles que les isoparaffines ISOPARS ou l'isododécane ou les huiles carbonées non-volatiles ;
- les silicones volatiles telles que les cyclométhicones ou les hexaméthyldisiloxanes ou les silicones non-volatiles.
La réaction sol/gel est catalysée en milieu acide ou en milieu basique, selon les propriétés du matériau final recherchées et l'utilisation envisagée.
La réaction d'hydrolyse/condensation pourra être effectuée par exemple à une température allant de 10 à 85°C et de préférence de 20 à 40°C.

Les précurseurs organométalliques sont choisis

dans le groupe constitué par
les silanes, les titanates ou zirconates répondant à l'une des formules suivantes :

   M(OR₁)₄ (Ia)

   R-M(OR₁)₃ (Ib)

   ₃(OR₁)―M―(OR₁)₃ (Ic)

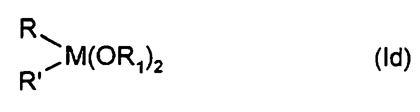

   dans lesquelles :
   M désigne Si, Ti ou Zr ;
   R₁ désigne un radical alkyle linéaire ou ramifié, de préférence en C₁-C₄ ;
   R et R' indépendamment l'un de l'autre, désignent un radical alkyle C₁-C₃₀ linéaire ou ramifié, un radical cycloalkyle C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy.

Une composition plus particulièrement préférée selon l'invention comprend au moins un colorant d'oxydation, au moins un système enzymatique tel que défini ci-dessus dont l'enzyme (oxydo-réductase à 2 ou à 4 électrons ou peroxydase ou leur mélange) est immobilisée dans une matrice de matériau obtenu par voie sol-gel, le matériau étant préparé à partir de tétraalcoxysilane, d'alkyltrialcoxysilane ou d'aminoalkyltrialcoxysilane ou les mélanges de ceux-ci, d'eau et d'acide chlorhydrique, que l'on mélange sous agitation à température ambiante, puis par ajout d'une solution aqueuse de la dite enzyme qui provoque en quelques minutes la formation d'un solide.

Le solide obtenu peut ensuite être broyé à la granulométire désirée.

Le ou les colorants d'oxydation utilisés dans la composition tinctoriale prête à l'emploi conforme à l'invention peuvent notamment être choisis parmi des bases d'oxydation et/ou des coupleurs.
Les bases d'oxydation sont notamment les paraphénylènediamines, les bases doubles, les para-aminophénols et les bases hétérocycliques.
A titre d'exemples, on peut citer parmi les paraphénylènesdiamines, celles de formule (IV) suivante, et leurs sels d'addition avec un acide : 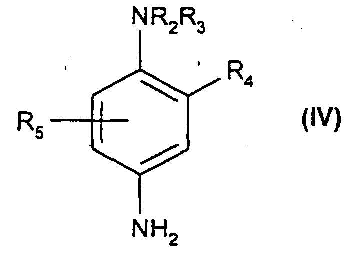 dans laquelle :
- R₂ et R₃, identiques ou différents, représentent un atome d'hydrogène, un radical alkyle en C₁-C₄, ou monohydroxyalkyle en C₁-C₄ ;
- R₄ représente un atome d'hydrogène ou d'halogène, un radical alkyle en C₁-C₄, ou monohydroxyalkyle en C₁-C₄ ;
- R₅ représente représentent un atome d'hydrogène ou un radical alkyle en C₁-C₄ ;

De préférence, les paraphénylènediamines de formule (IV) sont choisies parmi les suivantes :
la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, et leurs sels d'addition avec un acide.

Parmi les bases doubles utilisables à titre de base d'oxydation dans la composition tinctoriale prête à l'emploi conforme à l'invention, on peut notamment citer les composés comportant au moins deux noyaux aromatiques sur lesquels sont portés plusieurs groupements amino et/ou hydroxyle.

Parmi lesdites bases doubles on peut plus particulièrement citer les composés de formule (V) suivante, et leurs sels d'addition avec un acide : 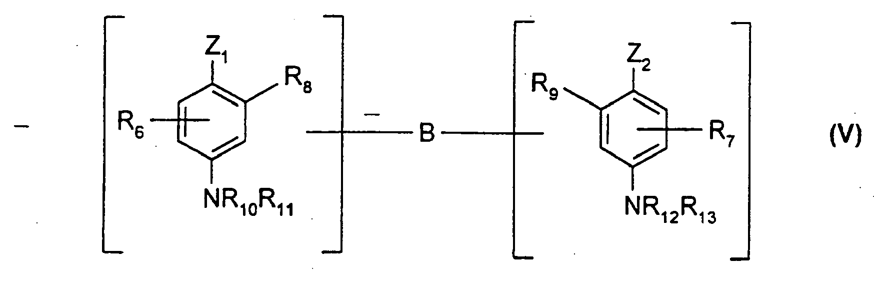 dans laquelle :
- Z₁ et Z₂, identiques ou différents, représentent un radical hydroxyle ou -NH₂ pouvant être substitué par un radical alkyle en C₁-C₄ ou par un bras de liaison B ;
- le bras de liaison B représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₆ et R₇ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison B ;
- R₈, R₉, R₁₀, R₁₁, R₁₂ et R₁₃, identiques ou différents, représentent un atome d'hydrogène, un bras de liaison B ou un radical alkyle en C₁-C₄ ;
étant entendu que les composés de formule (V) ne comportent qu'un seul bras de liaison B par molécule.

Parmi les groupements azotés de la formule (V) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.

Parmi les bases doubles de formule (V) ci-dessus, on peut plus particulièrement citer le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

Parmi ces bases doubles de formule (V), le N,N'-bis-(β-hydroxyéthyl) N,N'-bis-(4'-aminophényl) 1,3-diamino propanol, le 1,8-bis-(2,5-diaminophénoxy)-3,5-dioxaoctane ou l'un de leurs sels d'addition avec un acide sont particulièrement préférés.

Parmi les para-aminophénols utilisables à titre de base d'oxydation dans la composition tinctoriale prête à l'emploi conforme à l'invention, on peut notamment citer ceux de formule (VI) suivante et leurs sels d'addition avec un acide : 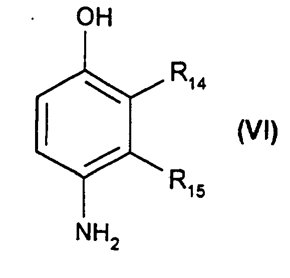 dans laquelle :
- R₁₄ et R₁₅, identiques ou différents, représentent un atome d'hydrogène ou d'halogène, un radical alkyle en C₁-C₄, monohydroxyalkyle en C₁-C₄, alcoxy(C₁-C₄)alkyle en C₁-C₄, aminoalkyle en C₁-C₄, monohydroxyalkyle(C₁-C₄)aminoalkyle en C₁-C₄,
étant entendu qu'au moins un des radicaux R₁₄ et R₁₅ représente un atome d'hydrogène ;

Parmi les para-aminophénols de formule (VI) décrite ci-dessus, on peut plus particulièrement citer le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les bases hétérocycliques utilisables à titre de base d'oxydation dans la composition tinctoriale prête à l'emploi conforme à l'invention, on peut plus particulièrement citer les dérivés pyridiniques, les dérivés pyrimidiniques, les dérivés pyrazoliques, et leurs sels d'addition avec un acide.
Parmi les dérivés pyridiniques, on peut plus particulièrement citer les composés décrits par exemple dans les brevets GB 1 026 978 et GB 1 153 196, comme la 2,5-diamino pyridine, la 2-(4-méthoxyphényl)amino 3-amino pyridine, la 2,3-diamino 6-méthoxy pyridine, la 2-(β-méthoxyéthyl)amino 3-amino 6-méthoxy pyridine, la 3,4-diamino pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demande de brevet WO 96/15765, comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl pyrazolo-[1,5-a]-pyrimidine-3,7-diamine ; la pyrazolo-[1,5-a]-pyrimidine-3,5-diamine ; la 2,7-diméthyl pyrazolo-[1,5-a]-pyrimidine-3,5-diamine ; le 3-amino pyrazolo-[1,5-a]-pyrimidin-7-ol ; le 3-amino pyrazolo-[1,5-a]-pyrimidin-5-ol ; le 2-(3-amino pyrazolo-[1,5-a]-pyrimidin-7-ylamino)-éthanol, le 2-(7-amino pyrazolo-[1,5-a]-pyrimidin-3-ylamino)-éthanol, le 2-[(3-amino-pyrazolo[1,5-a]pyrimidin-7-yl)-(2-hydroxy-éthyl)-amino]-éthanol, le 2-[(7-amino-pyrazolo[1,5-a]pyrimidin-3-yl)-(2-hydroxy-éthyl)-amino]-éthanol, la 5,6-diméthyl pyrazolo-[1,5-a]-pyrimidine-3,7-diamine, la 2,6-diméthyl pyrazolo-[1,5-a]-pyrimidine-3,7-diamine, la 2, 5, N 7, N 7-tetraméthyl pyrazolo-[1,5-a]-pyrimidine-3,7-diamine, et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino 1-méthyl pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition avec un acide.

Les coupleurs, sont notamment les méta-aminophénols, les méta-phénylènediamines, les métadiphénols, les naphtols, les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 2-méthyl-1-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

D'une manière générale, les sels d'addition avec un acide utilisables dans le cadre des compositions tinctoriales de l'invention (bases d'oxydation et coupleurs) sont notamment choisis parmi les chlorhydrates, les bromhydrates, les sulfates et les tartrates, les lactates et les acétates.

Selon l'invention, le ou les colorants d'oxydation représentent de préférence de 0,001 à 20 % en poids environ du poids total de la composition tinctoriale prête à l'emploi, et encore plus préférentiellement de 0,01 à 10 % en poids environ de ce poids.

Selon une forme de réalisation préférée, la composition tinctoriale prête à l'emploi conforme à l'invention peut en outre renfermer un ou plusieurs colorants directs notamment pour modifier les nuances en les enrichissant de reflets.

Le milieu approprié pour la teinture (ou support) de la composition tinctoriale prête à l'emploi conforme à l'invention est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale prête à l'emploi, et encore plus préférentiellement entre 5 et 30 % en poids environ.

Le pH de la composition prête à l'emploi conforme à l'invention est choisi de telle manière que l'activité enzymatique de l'oxydo-réductase à 2 électrons ou à 4 électrons ou de la peroxydase soit suffisante. Il est généralement compris entre 3 et 11 environ, et de préférence entre 4 et 9 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines, le 2-méthyl 2-amino 1-propanol ainsi que leurs dérivés, les hydroxydes de sodium ou de potassium et les composés de formule (VII) suivante : 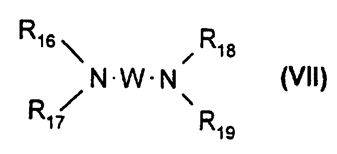 dans laquelle W est un reste propylène éventuellement substitué par un groupement hydroxyle ou un radical alkyle en C₁-C₄ ; R₁₆, R₁₇, R₁₈ et R₁₉, identiques ou différents, représentent un atome d'hydrogène, un radical alkyle en C₁-C₄ ou hydroxyalkyle en C₁-C₄.

La composition tinctoriale prête à l'emploi conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Bien entendu, l'homme de l'art veillera à choisir ce ou ces éventuels composés complémentaires de manière telle que les propriétés avantageuses attachées intrinsèquement à la composition tinctoriale prête à l'emploi conforme à l'invention ne soient pas, ou substantiellement pas, altérées par la ou les adjonctions envisagées.

La composition tinctoriale prête à l'emploi conforme à l'invention peut se présenter sous des formes diverses, telles que sous forme de liquides, de crèmes, de gels, éventuellement pressurisés, de mousses, ou sous toute autre forme appropriée pour réaliser une teinture des fibres kératiniques, et notamment des cheveux humains.
Dans le cas où les colorants d'oxydation et une ou plusieurs oxydoréductases à 2 électrons ou à 4 électrons sont présents au sein de la même composition prête à l'emploi, celle-ci peut être stockée avant utilisation à condition qu'elle soit exempte d'oxygène gazeux, de manière à éviter toute oxydation prématurée du ou des colorants d'oxydation.

L'invention a également pour objet un procédé de teinture des fibres kératiniques et en particulier des fibres kératiniques humaines telles que les cheveux mettant en oeuvre la composition tinctoriale prête à l'emploi telle que définie précédemment.

Selon ce procédé, on applique sur les fibres, à une température d'application comprise entre la température ambiante et 60°C, au moins une composition tinctoriale prête à l'emploi telle que définie précédemment, pendant un temps suffisant pour développer la coloration désirée. De façon préférentielle, les fibres sont ensuite rincées, éventuellement lavées au shampooing, puis séchées.

La température d'application est de préférence comprise entre la température ambiante et 45°C et encore plus préférentiellement entre 20°C et 37°C.

Le temps suffisant au développement de la coloration sur les fibres kératiniques est généralement compris entre 1 et 60 minutes et encore plus précisément entre 5 et 30 minutes.

De cette façon, l'un des procédés selon l'invention consiste à stocker à l'abri de l'air une composition comprenant , dans un milieu approprié pour la teinture, au moins un colorant d'oxydation (base et/ou coupleur) tel que défini précédemment et au moins un système enzymatique comprenant, soit (i) une oxydo-réductase à 4 électrons, soit (ii) une oxydoréductase à 2 électrons et au moins un donneur pour ladite oxydoréductase, soit (iii) une peroxydase et éventuellement un donneur pour ladite peroxydase, et une oxydoréducase à 2 électrons et un donneur pour ladite oxydoréductase,
la ou les enzymes étant immobilisées dans une matrice de matériau obtenu par un procédé sol-gel,
puis à appliquer ladite composition sur les fibres kératiniques en présence d'air.

Un autre procédé selon l'invention consiste à stocker à l'abri de l'air une matrice de matériau obtenu par voie sol-gel, contenant le mélange d'au moins un colorant d'oxydation et d'au moins un système enzymatique, ledit système comprenant, soit (i) une oxydo-réductase à 4 électrons, soit (ii) une oxydoréductase à 2 électrons et au moins un donneur pour ladite oxydoréductase, soit (iii) une peroxydase et éventuellement un donneur pour ladite peroxydase, et une oxydoréducase à 2 électrons et un donneur pour ladite oxydoréductase, à disperser ladite matrice dans un milieu approprié pour la teinture, puis à appliquer la composition obtenue sur les fibres kératiniques en présence d'air.

Selon une variante, le procédé consistant à stocker sous forme séparée, une composition (A), une composition B et éventuellement une composition C, définies dans les dispositifs décrits ci-après, puis à procéder à leur mélange au moment de l'emploi en présence d'air avant d'appliquer ce mélange sur les fibres kératiniques.

D'autres objets de l'invention sont des dispositifs de teinture à plusieurs compartiments ou "kit" de teinture ou tout autre système de conditionnement à plusieurs compartiments.

Un dispositif de teinture à 2 compartiments selon l'invention comprend un premier compartiment renfermant une composition A comprenant, dans un milieu approprié pour la teinture, au moins un colorant d'oxydation (base et/ou coupleur) tel que défini précédemment, et un second compartiment renfermant une composition B comprenant, dans un milieu approprié pour la teinture, au moins soit, (i) une oxydoréductase à 4 électrons, soit (ii) une oxydoréductase à 2 électrons, soit (iii) une peroxydase et une oxydoréductase à 2 électrons, la composition A ou la composition B contenant en outre au moins un donneur pour les cas de figure (ii) et (iii) où la composition contient une oxydoréductase à 2 électrons, lesdites enzymes étant immobilisées dans une matrice de matériau obtenu par un procédé sol-gel ; les compositions A et B pouvant également contenir un donneur pour la peroxydase dans le cas de figure (iii).

Un dispositif de teinture à 3 compartiments selon l'invention comprend un premier compartiment renfermant une composition A comprenant, dans un milieu approprié pour la teinture, au moins un colorant d'oxydation (base et/ou coupleur) tel que défini précédemment, un second compartiment renfermant une composition B comprenant, dans un milieu approprié pour la teinture, au moins une oxydoréductase à 2 électrons, un troisième compartiment renfermant une composition C comprenant, dans un milieu approprié pour la teinture, au moins un donneur pour l'oxydoréductase, la ou lesdites enzymes étant immobilisées dans une matrice de matériau obtenu par un procédé sol-gel.

Un autre dispositif de teinture à 3 compartiments selon l'invention comprend un premier compartiment renfermant une composition A comprenant, dans un milieu approprié pour la teinture, au moins un colorant d'oxydation (base et/ou coupleur) tel que défini précédemment, un second compartiment renfermant une composition B comprenant, dans un milieu approprié pour la teinture, au moins une peroxydase et au moins une oxydoréductase à 2 électrons, un troisième compartiment renfermant une composition C comprenant, dans un milieu approprié pour la teinture, au moins un donneur pour l'oxydoréductase ou un donneur pour la peroxydase,
la composition A ou la composition B pouvant contenir un ou plusieurs donneurs pour lesdites enzymes,
lesdites enzymes étant immobilisées dans une matrice de matériau obtenu par un procédé sol-gel.

Un dernier dispositif de teinture à 3 compartiments selon l'invention comprend un premier compartiment renfermant une composition A comprenant, dans un milieu approprié pour la teinture, au moins un colorant d'oxydation (base et/ou coupleur) tel que défini précédemment, un second compartiment renfermant une composition B comprenant, dans un milieu approprié pour la teinture, au moins une peroxydase, un troisième compartiment renfermant une composition C comprenant, dans un milieu approprié pour la teinture, au moins une oxydoréductase à 2 électrons, ou du peroxyde d'hydrogène,
la composition A et/ou la composition B et/ou la composition C contenant indifféremment les donneurs pour lesdites enzymes,
lesdites enzymes étant immobilisées dans une matrice de matériau obtenu par un procédé sol-gel.
Pour ce dernier dispositif, lorsque la composition C contient du peroxyde d'hydrogène, les donneurs du ou des enzymes sont préférentiellement présents dans les compositions A et B.

Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLE 1 : Préparation d'Uricase immobilisée

On a mélangé 8 grammes de tétraméthoxysilane avec 3 grammes d'eau et 0,2 grammes d'une solution d'acide chlorhydrique 0.04 Molaire.
Après vingt minutes d'agitation à la température ambiante, on a obtenu une solution homogène.
A cette solution on a rajouté 11,2 grammes d'une solution aqueuse d'uricase d'Arthrobacter Globiformis à 20 U.I. /mg commercialisée par la société SIGMA ayant 1.10⁶ U%.
Après quelques minutes on a obtenu un solide dur et cassant.
Le solide obtenu a été broyé, lavé avec de l'eau permutée puis séché, pour obtenir une poudre cristalline.

### EXEMPLE 2 : Préparation de Laccase immobilisée

On a mélangé 40 grammes de tétraméthoxysilane avec 0,5 grammes d'une solution d'acide chlorhydrique 0.04 Molaire.
Après vingt minutes d'agitation à la température ambiante, on a obtenu une solution homogène.
A cette solution on a rajouté une solution aqueuse de laccase SP809 à 10.10⁶ unités U commercialisée par la société NOVO NORDISK ayant 6.10⁸ U%. Après quelques minutes on a obtienu un solide dur et cassant.
Le solide obtenu a été broyé, lavé avec de l'eau permutée puis séché, pour obtenir une poudre cristalline.

### EXEMPLE 3 : Composition tinctoriale

On a préparé la composition de teinture suivante :

| | |
|---|---|
| Paraphénylènediamine 2HCl | 1,0 g |
| N-acétylcystéine | 0,10 g |
| 6-méthoxy-benzo[1,3]dioxol-5-ylamine HCl | 0,71 g |
| 1-β-hydroxyéthyloxy-2,4-diaminobenzène 2HCl | 0,88 g |
| Uricase immobilisée de l'exemple 1 | 4,0 g |
| Acide urique | 1,0 g |
| Monooléate de polyglycérol vendu sous la dénomination Decaglyn 1-0 par la société NIKKO | 1,0 g |
| Aculyn 22 de la société ROHM et HAAS* | 0,75 gMA** |
| 2-amino-2-méthyl-1-propanol qs pH | 9,5 |
| Eau déminéralisée q.s.p. | 100 g |

| | |
|---|---|
| * Terpolymère acide méthacrylique/acrylate d'éthyle/méthacrylate de stéaryle oxyéthyléné (55/35/10)en dispersion aqueuse à 30% | |
| ** désigne Matière Active | |

Cette composition a été préparée au moment de l'emploi par mélange des 4 grammes d'uricase immobilisée au reste de la composition et a été appliquée sur des cheveux gris naturels à 90% de blancs.
Après 30 minutes de pause, les cheveux ont été rincés, lavés avec un shampooing standard puis séchés.
Les cheveux ont été teints dans une nuance noire intense.

### EXEMPLE 4 : Composition tinctoriale

On a préparé la composition de teinture suivante :

| | |
|---|---|
| 4-aminophénol | 0,11 g |
| N-acétylcystéine | 0,10 g |
| 2-méthyl-5-aminophénol | 0,12 g |
| Uricase immobilisée de l'exemple 1 | 4,0 g |
| Acide urique | 1,0 g |
| Monooléate de polyglycérol vendu sous la dénomination Decaglyn 1-0 par la société NIKKO | 1,0 g |
| Aculyn 22 de la société ROHM et HAAS* | 0,75 gMA** |
| 2-amino-2-méthyl-1-propanol qs pH | 9,5 |
| Eau déminéralisée q.s.p. | 100 g |

| | |
|---|---|
| * Terpolymère acide méthacrylique/acrylate d'éthyle/méthacrylate de stéaryle oxyéthyléné (55/35/10)en dispersion aqueuse à 30% | |
| ** désigne Matière Active | |

Cette composition a été préparée au moment de l'emploi par mélange des 4 grammes d'uricase immobilisée au reste de la composition et a été appliquée sur des cheveux gris naturels à 90% de blancs.
Après 30 minutes de pause, les cheveux ont été rincés, lavés avec un shampooing standard puis séchés .
Les cheveux ont été teints dans une nuance doré-cuivré intense.

### EXEMPLE 5 : Composition tinctoriale

On a préparé la composition de teinture suivante :

| | |
|---|---|
| Paraphénylènediamine 2HCl | 0,18 g |
| N-acétylcystéine | 0,10 g |
| 6-hydroxybenzomorpholine | 0,15 g |
| Laccase immobilisée de l'exemple 2 | 2,8 g |
| Monooléate de polyglycérol vendu sous la dénomination Decaglyn 1-0 par la société NIKKO | 1,0 g |
| Aculyn 22 de la société ROHM et HAAS* | 0,75 gMA** |
| 2-amino-2-méthyl-1-propanol qs pH | 7 |
| Eau déminéralisée q.s.p. | 100 g |

| | |
|---|---|
| * Terpolymère acide méthacrylique/acrylate d'éthyle/méthacrylate de stéaryle oxyéthyléné (55/35/10)en dispersion aqueuse à 30% | |
| ** désigne Matière Active | |

Cette composition a été préparée au moment de l'emploi par mélange des 2,8 grammes de laccase immobilisée au reste de la composition et a été appliquée sur des cheveux gris naturels à 90% de blancs.
Après 30 minutes de pause, les cheveux ont été rincés, lavés avec un shampooing standard puis séchés .
Les cheveux ont été teints dans une nuance naturel-cendré intense.

### EXEMPLE 6 : Composition tinctoriale

On a préparé la composition de teinture suivante :

| | |
|---|---|
| Paraphénylènediamine 2HCl | 0,18 g |
| N-acétylcystéine | 0,10 g |
| 2-méthyl-5-aminophénol | 0,12 g |
| Laccase immobilisée de l'exemple 2 | 2,8 g |
| Monooléate de polyglycérol vendu sous la dénomination Decaglyn 1-0 par la société NIKKO | 1,0 g |
| Aculyn 22 de la société ROHM et HAAS* | 0,75 gMA** |
| 2-amino-2-méthyl-1-propanol qs pH | 7 |
| Eau déminéralisée q.s.p. | 100 g |

| | |
|---|---|
| * Terpolymère acide méthacrylique/acrylate d'éthyle/méthacrylate de stéaryle oxyéthyléné (55/35/10)en dispersion aqueuse à 30% | |
| ** désigne Matière Active | |

Cette composition a été préparée au moment de l'emploi par mélange des 2,8 grammes de laccase immobilisée au reste de la composition et a été appliquée sur des cheveux gris naturels à 90% de blancs.
Après 30 minutes de pause, les cheveux ont été rincés, lavés avec un shampooing standard puis séchés .
Les cheveux ont été teints dans une nuance violine.

## Revendications

1. Composition prête à l'emploi, pour la teinture d'oxydation des fibres kératiniques et en particulier des fibres kératiniques humaines telles que les cheveux, comprenant dans un milieu approprié pour la teinture, au moins un colorant d'oxydation et au moins un système enzymatique choisi parmi :
(a) au moins une oxydoréductase à 2 électrons associée à un donneur,
(b) au moins une oxydoréductase à 4 électrons,
(c)
(i) au moins une peroxydase éventuellement associée à un donneur et
(ii) du peroxyde d'hydrogène, ou un système enzymatique le générant in situ,
**caractérisée par le fait que** la ou lesdites enzymes sont immobilisées dans une matrice de matériau obtenu par hydrolyse acide, ou basique partielle ou totale, de précurseurs organométalliques, puis polycondensation, les précurseurs organométalliques étant des oxydes métalliques choisis dans le groupe formé par :
(1) les silanes, les titanates ou zirconates répondant à l'une des formules suivantes :
M(OR₁)₄ (Ia)
R-M(OR₁)₃ (Ib)
₃(OR₁)-M-(OR₁)₃ (Ic) 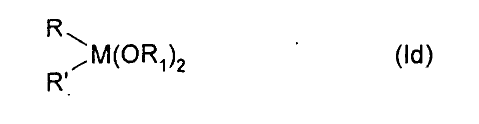
dans lesquelles :
M désigne Si, Ti ou Zr ;
R₁ désigne un radical alkyle linéaire ou ramifie, de préférence en C₁-C₄ ;
R et R' indépendamment l'un de l'autre, désignent un radical alkyle C₁-C₃₀ linéaire ou ramifié, un radical cycloalkyle C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy.
N (OR₁)₍₄₋ₜ₎(X)ₜ (IIIa)
et leurs mélanges.

2. Composition selon l'une quelconque des revendications 1 à 3, **caractérisée par le fait que** le matériau est obtenu à température ambiante par hydrolyse acide ou basique et condensation de tétraalcoxysilane, d'alkyltrialcoxysilane ou d'aminoalkyltrialcoxysilane ou leurs mélanges par réaction sol-gel.

3. Composition selon l'une quelconque des revendications 1 ou 2, **caractérisée par le fait que** la ou les oxydo-réductases à 2 électrons sont utilisées avec un donneur pour la ou lesdites enzymes et sont choisies parmi les pyranose oxydases, les glucose oxydases, les glycérol oxydases, les lactate oxydases, les pyruvate oxydases, les uricases, les choline oxydases, les sarcosine oxydases, les bilirubine oxydases et les aminoacides oxydases.

4. Composition selon l'une quelconque des revendications 1 à 3, **caractérisée par le fait que** les oxydo-réductases à 4 électrons sont choisis parmi les laccases, les tyrosinases, les catéchol oxydases et les polyphénols oxydases.

5. Composition selon la revendication 4, **caractérisée par le fait que** les oxydo-réductases à 4 électrons sont choisies parmi les laccases d'origine végétale, d'origine animale, d'origine fongique ou d'origine bactérienne et parmi les laccases obtenues par biotechnologie.

6. Composition selon la revendication 5, **caractérisée par le fait que caractérisée par le fait que** la laccase est d'origine végétale et choisie parmi les laccases présentes dans les extraits d'Anacardiacées ; de Podocarpacées ; de Rosmarinus off. ; de Solanum tuberosum ; d'Iris sp. ; de Coffea sp. ; de Daucus carrota ; de Vinca minor ; de Persea americana ; de Catharenthus roseus ; de Musa sp. ; de Malus pumila ; de Gingko biloba ; de Monotropa hypopithys (sucepin), d'Aesculus sp. ; d'Acer pseudoplatanus ; de Prunus persica et de Pistacia palaestina.

7. Composition selon la revendication 5, **caractérisée par le fait que** la laccase est d'origine fongique ou obtenue par biotechnologie.

8. Composition selon la revendication 7, **caractérisée par le fait que** la laccase est choisie parmi les laccases issues de Polyporus versicolor, de Rhizoctonia praticola, de Rhus vernicifera, de Scytalidium, de Polyporus pinsitus, de Myceliophtora thermophila, de Rhizoctonia solani, de Pyricularia orizae, de Trametes versicolor, de Fomes fomentarius, de Chaetomium thermophile, de Neurospora crassa, de Colorius versicol, de Botrytis cinerea, de Rigidoporus lignosus, de Phellinus noxius, de Pleurotus ostreatus, d'Aspergillus nidulans, de Podospora anserina, d'Agaricus bisporus, de Ganoderma lucidum, de Glomerella cingulata, de Lactarius piperatus, de Russula delica, d'Heterobasidion annosum, de Thelephora terrestris, de Cladosporium cladosporioides, de Cerrena unicolor, de Coriolus hirsutus, de Ceriporiopsis subvermispora, de Coprinus cinereus, de Panaeolus papilionaceus, de Panaeolus sphinctrinus, de Schizophyllum commune, de Dichomitius squalens, et de leurs variantes.

9. Composition selon l'une quelconque des revendications précédentes, **caractérisée par le fait que** les oxydo-réductases à 2 électrons ou à 4 électrons représentent de 0,01 à 20 % en poids du poids total de la composition tinctoriale prête à l'emploi.

10. Composition selon la revendication 9, **caractérisée par le fait que** les oxydo-réductases à 2 électrons ou à 4 électrons représentent de 0,1 à 10% en poids du poids total de la composition tinctoriale prête à l'emploi.

11. Composition selon l'une quelconque des revendications 1 ou 2, **caractérisée par le fait que** la ou les peroxydases sont des peroxydases simplex.

12. Composition selon l'une quelconque des revendications 1 ou 2, **caractérisée par le fait que** la ou les peroxydases sont des catalases.

13. Composition selon l'une quelconque dés revendications 1 ou 2, **caractérisée par le fait que** la ou les peroxydases sont utilisées avec un donneur pour la ou lesdites enzymes et sont choisies parmi les NADH peroxydases, les acides gras peroxydases, les NADPH peroxydases, les cytochrome-c peroxydases, les iodures peroxydases, les chlorures peroxydases, les L. ascorbates, les glutathion peroxydases.

14. Composition selon l'une quelconque des revendications 1 ou 2, **caractérisée par le fait que** la ou les peroxydases sont d'origine animale, végétale, fongique, bactérienne ou obtenues par biotechnologie.

15. Composition selon la revendication 14, **caractérisée par le fait que** la ou les peroxydases sont issues de la pomme, de l'abricot, de forge, du radis noir, de la betterave, du chou, de la carotte, du maïs, du coton, de l'ail, du raisin, de la menthe, de la rhubarbe, du soja, de l'épinard, du coprin, du lait de bovin, des microorganismes du type Acetobacter peroxidans, Staphylococcus faecalis, Arthromycesramosus.

16. Composition selon l'une quelconque des revendications 11 à 15, **caractérisée par le fait que** la ou les peroxydases représentent de 0,0001 à 20% en poids du poids total de la composition tinctoriale prête à l'emploi .

17. Composition selon la revendication 16, **caractérisée par le fait que** la ou les peroxydases représentent de 0,001 à 10% en poids du poids total de la composition tinctoriale prête à l'emploi-.

18. Composition selon l'une quelconque des revendications précédentes, **caractérisée par le fait que** le ou les colorants d'oxydation sont choisis parmi les bases d'oxydation et/ou les coupleurs.

19. Composition la revendication 18, **caractérisée par le fait que** les bases d'oxydation sont des paraphénylènediamines, des bases doubles, des para-aminophénols et des bases hétérocycliques, et leurs sels d'addition avec un acide.

20. Composition selon la revendication 19, **caractérisée par le fait que** les paraphénylènediamines sont choisies parmi celles de formule (IV) suivante et leurs sels d'addition avec un acide : 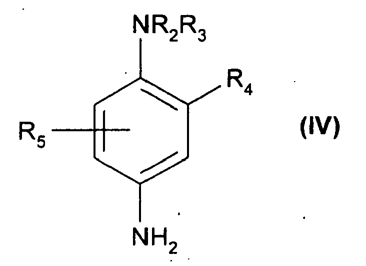 dans laquelle :
- R₂ et R₃, identiques ou différents, représentent un atome d'hydrogène, un radical alkyle en C₁-C₄, ou monohydroxyalkyle en C₁-C₄ ;
- R₄ représente un atome d'hydrogène ou d'halogène, un radical alkyle en C₁-C₄, ou monohydroxyalkyle en C₁-C₄ ;
- R₅ représente représentent un atome d'hydrogène ou un radical alkyle en C₁-C₄ ;

21. Composition selon la revendication 20, **caractérisée par le fait que** les paraphénylènediamines de formule (IV) sont choisies parmi :
la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl'paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, et leurs sels d'addition avec un acide.

22. Composition selon la revendication 19, **caractérisée par le fait que** les bases doubles sont choisies parmi les composés de formule (V) suivante, et leurs sels d'addition avec un acide : 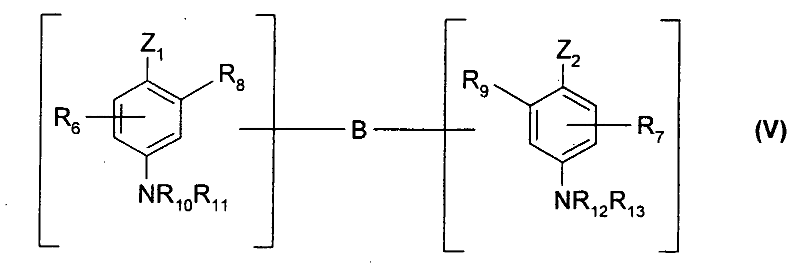 dans laquelle :
- Z₁ et Z₂; identiques ou différents, représentent un radical hydroxyle ou -NH₂ pouvant être substitué par un radical alkyle en C₁-C₄ ou par un bras de liaison B ;
- le bras de liaison B représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
- R₆ et R₇ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison B ;
- R₈, R₉, R₁₀, R₁₁, R₁₂ et R₁₃, identiques ou différents, représentent un atome d'hydrogène, un bras de liaison B ou un radical alkyle en C₁-C₄ ; étant entendu que les composés de formule (V) ne comportent qu'un seul bras de liaison B par molécule.

23. Composition selon la revendication 20, **caractérisée par le fait que** les bases doubles de formule (V) sont choisies parmi le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diaminophénoxy)-3,5-dioxaoctane, et leurs sels d'addition avec un acide.

24. Composition selon la revendications 19, **caractérisée par le fait que** les para-aminophénols sont choisis parmi ceux de formule (VI) suivante et leurs d'addition avec un acide : 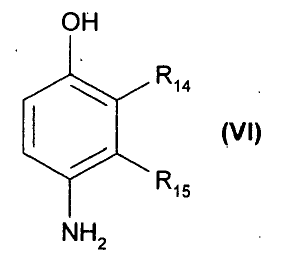 dans laquelle :
- R₁₄ et R₁₅, identiques ou différents, représentent un atome d'hydrogène ou d'halogène, un radical alkyle en C₁-C₄, monohydroxyalkyle en C₁-C₄, alcoxy(C₁-C₄)alkyle en C₁-C₄, aminoalkyle en C₁-C₄, monohydroxyalkyle(C₁-C₄)aminoalkyle en C₁-C₄,
étant entendu qu'au moins un des radicaux R₁₄ et R₁₅ représente un atome d'hydrogène.

25. Composition selon la revendication 24, **caractérisée par le fait que** les para-aminophénols de formule (VI) sont choisis parmi le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

26. Composition selon la revendication 19, **caractérisée par le fait que** les bases hétérocycliques sont choisies parmi les dérivés pyridiniques, les dérivés pyrimidiniques dont les pyrazolopyrimidines, les dérivés pyrazoliques, et leurs sels d'addition avec un acide.

27. Composition selon la revendication 19, **caractérisée par le fait que** les coupleurs sont des méta-aminophénols, des métaphénylènediamines, des métadiphénols, des naphtols, ou dés coupleurs hétérocycliques, et leurs sels d'addition avec un acide.

28. Composition selon la revendication 27, **caractérisée par le fait que** les coupleurs sont choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 2-méthyl-1-naphtol, lé 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, et leurs sels d'addition avec un acide.

29. Composition selon l'une quelconque des revendications 18 à 28, **caractérisée par le fait que** le ou les colorants d'oxydation (bases et/ou coupleurs) représentent de 0,001 à 20 % en poids du poids total de la composition tinctoriale prête à l'emploi.

30. Composition selon la revendication 29, **caractérisée par le fait que** le ou les colorants d'oxydation représentent de 0,01 à 10 % en poids du poids total de la composition tinctoriale prête à l'emploi.

31. Composition selon l'une quelconque des revendications précédentes, **caractérisée par le fait qu'**elle renferme un ou plusieurs colorants directs.

32. Composition selon l'une quelconque des revendications précédentes, **caractérisée par le fait qu'**elle présente un pH compris entre 3 et 11.

33. Composition selon l'une quelconque des revendications précédentes, **caractérisée par le fait que** le milieu approprié pour la teinture est l'eau ou un mélange d'eau et d'au moins un solvant organique.

34. Procédé de teinture d'oxydation des fibres kératiniques et en particulier des fibres kératiniques humaines telles que les cheveux, **caractérisé par le fait qu'**on applique sur lesdites fibres, en présence d'air, et à une température d'application comprise entre la température ambiante et 60°C, et pendant un temps suffisant pour développer la coloration désirée, une composition tinctoriale stockée à l'abri de l'air et comprenant, dans un milieu approprié pour la teinture, au moins un colorant d'oxydation (base et/ou coupleur) tel que défini à l'une quelconque des revendications 18-30, et au moins un système enzymatique comprenant, soit (i) une oxydo-réductase à 4 électron, soit (ii) une oxydoréductase à 2 électrons et au moins un donneur pour ladite oxydoréductase, soit (iii) une peroxydase et éventuellement un donneur pour ladite peroxydase, et une oxydoréductase à 2 électrons et un donneur pour ladite oxydoréductase, les enzymes étant immobilisées dans une matrice de matériau obtenu par hydrolyse acide, ou basique partielle ou totale, de précurseurs organométalliques, puis polycondensation, ;les précurseurs organométalliques étant des oxydés métalliques choisis dans le groupe formé par (1) les silanes, les titanates ou zirconates répondant à l'une des formules suivantes :
M(OR₁)₄ (Ia)
R-M(OR₁)₃ (Ib)
₃(OR₁)-M-(OR₁)₃ (Ic) 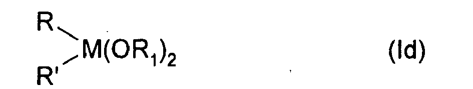
dans lesquelles :
M désigne Si, Ti ou Zr ;
R₁ désigne un radical alkyle linéaire ou ramifié, de préférence en C₁₋C₄,
R et R' indépendamment l'un de l'autre, désignent un radical alkyle C₁-C₃₀ linéaire ou ramifié, un radical cycloalkyle C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy, et leurs mélanges.

35. Procédé selon la revendication 34, **caractérisé par le fait que** la température d'application est comprise entre la température ambiante et 45°C.

36. Procédé selon les revendications 34 ou 35, **caractérisé par le fait que** le temps suffisant au développement de la coloration est compris entre 1 et 60 minutes.

37. Procédé selon la revendication 36, **caractérisé par le fait que** le temps suffisant au développement de la coloration est compris entre 5 et 30 minutes.

38. Dispositif de teinture à deux compartiments, **caractérisé par le fait qu'**il comporte un premier compartiment renfermant une composition A comprenant, dans un milieu approprié pour la teinture, au moins un colorant d'oxydation (base et/ou coupleur) tel que défini à l'une quelconque des revendications 18-30, et un second compartiment renfermant une composition B comprenant, dans un milieu approprié pour la teinture, au moins soit, (i) une oxydoréductase à 4 électrons, soit (ii) une oxydoréductase à 2 électrons, soit (iii) une peroxydase et une oxydoréductase à 2 électrons, la composition A ou la composition B contenant en outre au moins un donneur pour les cas de figure (ii) et (iii) où la composition contient une oxydoréductase à 2 électrons, et les compositions A et B pouvant contenir un donneur pour la peroxydase dans le cas de figure (iii), et lesdites enzymes étant immobilisées dans une matrice de matériau obtenu par hydrolyse acide, ou basique partielle ou totale, de précurseurs organométalliques, puis polycondensation, les précurseurs organométalliques étant des oxydes métalliques choisis dans le groupe formé par (1) les silanes, les titanates ou zirconates répondant à l'une des formules suivantes :
M(OR₁)₄ (Ia)
R-M(OR₁)₃ (Ib)
₃(OR₁)-M-(OR₁)₃ (Ic) 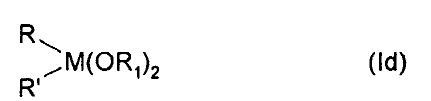
dans lesquelles :
M désigne Si, Ti ou Zr ;
R₁ désigne un radical alkyle linéaire ou ramifie, de préférence en C₁-C₄ ;
R et R' indépendamment l'un de l'autre, désignent un radical alkyle C₁-C₃₀ linéaire ou ramifié, un radical cycloalklye C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy, et lerus mélanges.

39. Dispositif de teinture à 3 compartiments comprenant un premier compartiment renfermant une composition A comprenant, dans un milieu approprié pour la teinture, au moins un colorant d'oxydation (base et/ou coupleur) tel que défini à l'une quelconque des revendications 18-30, un second compartiment renfermant une composition B comprenant, dans un milieu approprié pour la teinture, au moins une oxydoréductase à 2 électrons, un troisième compartiment renfermant une composition C comprenant, dans un milieu approprié pour la teinture, au moins un donneur pour l'oxydoréductase, et lesdites enzymes étant immobilisées dans une matrice de matériau obtenu par hydrolyse acide, ou basique partielle ou totale, de précurseurs organométalliques puis polycondensation, les précurseurs organométalliques étant des oxydes métalliques choisis dans le groupe formé par (1) les silanes les titanates ou zirconates répondant à l'une des formules suivantes :
M(OR₁)₄ (Ia)
R-M(OR₁)₃ (Ib)
₃(OR₁)-M-(OR₁)₃ (Ic) 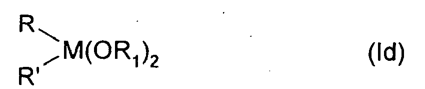
dans lesquelles:
M désigne Si, Ti ou Zr ;
R₁ désigne un radical alkyle linéaire ou ramifié, de préférence en C₁-C₄ ;
R et R' indépendamment l'un de l'autre, désignent un radical alkyle C₁-C₃₀ linéaire ou ramifié, un radical cycloalkyle C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy, et leurs mélanges.

40. Dispositif de teinture à 3 compartiments comprenant un premier compartiment renfermant une composition A comprenant, dans un milieu approprié pour la teinture, au moins un colorant d'oxydation (base et/ou coupleur) tel que défini à l'une quelconque des revendications 18-30, un second compartiment renfermant une composition B comprenant, dans un milieu approprié pour la teinture, au moins une peroxydase et au moins une oxydoréductase à 2 électrons, un troisième compartiment renfermant une composition C comprenant, dans un milieu approprié pour la teinture, au moins un donneur pour l'oxydoréductase ou un donneur pour la peroxydase,
la composition A ou la composition B pouvant contenir un ou plusieurs donneurs pour lesdites enzymes,
et lesdites enzymes étant immobilisées dans une matrice de matériau obtenu par hydrolyse acide, ou basique partielle ou totale, de précurseurs organométalliques, puis polycondensation, les précurseurs organométalliques étant des oxydes métalliques choisis dans le groupe formé par (1) les silanes, les titanates ou zirconates répondant à l'une des formules suivantes :
M(OR₁)₄ (Ia)
R-M(OR₁)₃ (Ib)
₃(OR₁)-M-(OR₁)₃ (Ic) 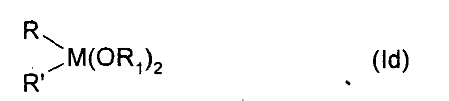
dans lesquelles :
M désigne Si, Ti ou Zr ;
R₁ désigne un radical alkyle linéaire ou ramifié, de préférence en C₁-C₄ :
R et R' indépendamment l'un de l'autre, désignent un radical alkyle C₁-C₃₀ linéaire ou ramifié, un radical cycloalkyle C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy.

41. Dispositif de teinture à 3 compartiments comprenant un premier compartiment renfermant une composition A comprenant, dans un milieu approprié pour la teinture, au moins un colorant d'oxydation (base et/ou coupleur) tel que défini à l'une quelconque des revendications 18-30, un second compartiment renfermant une composition B comprenant, dans un milieu approprié pour la teinture, au moins une peroxydase, un troisième compartiment renfermant une composition C comprenant, dans un milieu approprié pour la teinture, au moins une oxydoréductase à 2 électrons ou du peroxyde d'hydrogène,
la composition A et/ou la composition B et/ou la composition C contenant indifféremment les donneurs pour lesdites enzymes,
et lesdites enzymes étant immobilisées dans une matrice de matériau obtenu par hydrolyse acide ou basique partielle ou totale, de précurseurs organométalliques, puis polycondensation les précurseurs organométalliques étant des oxydes métalliques choisis dans le groupe formé par (1) les silanes, les titanates ou zirconates répondant à l'une des formules suivantes :
M(OR₁)₄ (Ia)
R-M(OR₁)₃ (Ib)
₃(OR₁)-M-(OR₁)₃ (Ic) 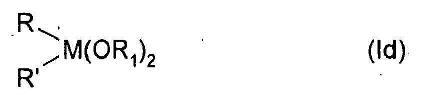
dans lesquelles :
M désigne Si, Ti ou Zr;
R₁ désigne un radical alkyle linéaire ou ramifié, de préférence en C₁-C₄;
R et R' indépendamment l'un de l'autre, désignent un radical alkyle C₁-C₃₀ linéaire ou ramifié, un radical cycloalkyle C₁-C₃₀, un radical aryle, ou aralkyle C₁-C₃₀ ou alkylC₁-C₃₀aryle substitué ou non, lesdits radicaux R et R' pouvant être substitués par un ou plusieurs groupes amino, carboxy ou hydroxy, et lerus mélanges.

42. Procédé selon l'une quelconque des revendications 34 à 37, **caractérisé par le fait qu'**il comporte une étape préliminaire consistant à stocker sous forme séparée, une composition A, une composition B, et éventuellement une composition C telles que définies à l'une quelconque des revendications 38-41, puis à procéder à leur mélange au moment de l'emploi en présence d'air, avant d'appliquer ce mélange sur les fibres kératiniques.

## Claims

1. Ready-to-use composition for the oxidation dyeing of keratin fibres, and in particular of human keratin fibres such as the hair, comprising, in a medium which is suitable for dyeing, at least one oxidation dye and at least one enzymatic system chosen from:
(a) at least one 2-electron oxidoreductase combined with a donor,
(b) at least one 4-electron oxidoreductase,
(c)
(i) at least one peroxidase optionally combined with a donor, and
(ii) hydrogen peroxide or an enzymatic system generating it in situ,
**characterized in that** the said enzyme(s) is (are) immobilized in a matrix of material obtained by acidic hydrolysis, partial or total basic hydrolysis of organometallic precursors, followed by polycondensation, the organometallic precursors being metal oxides chosen from the group formed by:
(1) silanes, titanates or zirconates corresponding to one of the following formulae:
M(OR₁)₄ (Ia)
R-M(OR₁)₃ (Ib)
₃(OR₁)-M-(OR₁)₃ (Ic) 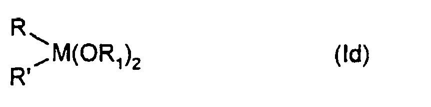
in which:
M denotes Si, Ti or Zr;
R₁ denotes a linear or branched, preferably C₁-C₄, alkyl radical;
R and R', independently of each other, denote a linear or branched C₁-C₃₀ alkyl radical, a C₁-C₃₀ cycloalkyl radical or an aryl or C₁-C₃₀ aralkyl or (C₁-C₃₀)-alkylaryl radical which may be substituted or unsubstituted, the said radicals R and R' possibly being substituted with one or more amino, carboxyl or hydroxyl groups, and mixtures thereof.

2. Composition according to Claim 1, **characterized in that** the material is obtained at room temperature by acidic or basic hydrolysis and condensation of tetraalkoxysilane, alkyltrialkoxysilane or aminoalkyltrialkoxysilane or mixtures thereof via a sol-gel reaction.

3. Composition according to either of Claims 1 and 2, **characterized in that** the 2-electron oxidoreductase(s) is(are) used with a donor for the said enzyme(s) and is(are) chosen from pyranose oxidases, glucose oxidases, glycerol oxidases, lactate oxidases, pyruvate oxidases, uricases, choline oxidases, sarcosine oxidases, bilirubin oxidases and amino acid oxidases.

4. Composition according to any one of Claims 1 to 3, **characterized in that** the 4-electron oxidoreductases are chosen from laccases, tyrosinases, catechol oxidases and polyphenol oxidases.

5. Composition according to Claim 4, **characterized in that** the 4-electron oxidoreductases are chosen from the laccases of plant origin, of animal origin, of fungal origin or of bacterial origin and from laccases obtained by biotechnology.

6. Composition according to Claim 5, **characterized in that** the laccase is of plant origin and is chosen from laccases present in extracts of Anacardiacea plants; of Podocarpacea plants; of Rosmarinus off.; of Solanum tuberosum; of Iris sp.; of Coffea sp.; of Daucus carrota; of Vinca minor; of Persea americana; of Catharanthus roseus; of Musa sp.; of Malus pumila; of Gingko biloba; of Monotropa hypopithys (Indian pipe), of Aesculus sp.; of Acer pseudoplatanus; of Prunus persica and of Pistacia palaestina.

7. Composition according to Claim 5, **characterized in that** the laccase is of fungal origin or is obtained by biotechnology.

8. Composition according to Claim 7, **characterized in that** the laccase is chosen from the laccases obtained from Polyporus versicolor, from Rhizoctonia praticola, from Rhus vernicifera, from Scytalidium, from Polyporus pinsitus, from Myceliophthora thermophila, from Rhizoctonia solani, from Pyricularia orizae, from Trametes versicolor, from Fomes fomentarius, from Chaetomium thermophile, from Neurospora crassa, from Colorius versicol, from Botrytis cinerea, from Rigidoporus lignosus, from Phellinus noxius, from Pleurotus ostreatus, from Aspergillus nidulans, from Podospora anserina, from Agaricus bisporus, from Ganoderma lucidum, from Glomerella cingulata, from Lactarius piperatus, from Russula delica, from Heterobasidion annosum, from Thelephora terrestris, from Cladosporium cladosporioides, from Cerrena unicolor, from Coriolus hirsutus, from Ceriporiopsis subvermispora, from Coprinus cinereus, from Panaeolus papilionaceus, from Panaeolus sphinctrinus, from Schizophyllum commune, from Dichomitius squalens, and from variants thereof.

9. Composition according to any one of the preceding claims, **characterized in that** the 2-electron or 4-electron oxidoreductases represent from 0.01% to 20% by weight relative to the total weight of the ready-to-use dye composition.

10. Composition according to Claim 9, **characterized in that** the 2-electron or 4-electron oxidoreductases represent from 0.1% to 10% by weight relative to the total weight of the ready-to-use dye composition.

11. Composition according to either of Claims 1 and 2, **characterized in that** the peroxidase(s) is(are) simplex peroxidases.

12. Composition according to either of Claims 1 and 2, **characterized in that** the peroxidase(s) is(are) catalases.

13. Composition according to either of Claims 1 and 2, **characterized in that** the peroxidase(s) is(are) used with a donor for the said enzyme(s) and are chosen from NADH peroxidases, fatty acid peroxidases, NADPH peroxidases, cytochrome-c peroxidases, iodide peroxidases, chloride peroxidases, L-ascorbates and glutathione peroxidases.

14. Composition according to either of Claims 1 and 2, **characterized in that** the peroxidase(s) is(are) of animal, plant, fungal or bacterial origin or are obtained by biotechnology.

15. Composition according to Claim 14, **characterized in that** the peroxidase(s) is(are) obtained from apple, apricot, barley, black radish, beetroot, cabbage, carrot, corn, cotton, garlic, grape, mint, rhubarb, soybean, spinach, ink cap, cow's milk or microorganisms such as Acetobacter peroxidans, Staphylococcus faecalis or Arthromycesramosus.

16. Composition according to any one of Claims 11 to 15, **characterized in that** the peroxidase(s) represent(s) from 0.0001% to 20% by weight relative to the total weight of the ready-to-use dye composition.

17. Composition according to Claim 16, **characterized in that** the peroxidase(s) represent(s) from 0.001% to 10% by weight relative to the total weight of the ready-to-use dye composition.

18. Composition according to any one of the preceding claims, **characterized in that** the oxidation dye(s) is(are) chosen from oxidation bases and/or couplers.

19. Composition according to Claim 18, **characterized in that** the oxidation bases are para-phenylenediamines, double bases, para-aminophenols and heterocyclic bases, and the addition salts thereof with an acid.

20. Composition according to Claim 19, **characterized in that** the para-phenylenediamines are chosen from those of formula (IV) below, and the addition salts thereof with an acid: 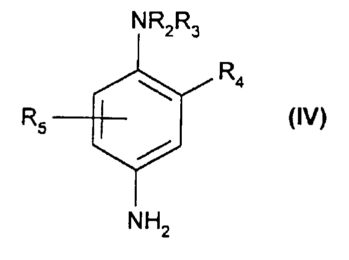 in which:
- R₂ and R₃, which may be identical or different, represent a hydrogen atom, a C₁-C₄ alkyl radical or a C₁-C₄ monohydroxyalkyl radical;
- R₄ represents a hydrogen or halogen atom, a C₁-C₄ alkyl radical or a C₁-C₄ monohydroxyalkyl radical;
- R₅ represents a hydrogen atom or a C₁-C₄ alkyl radical.

21. Composition according to Claim 20, **characterized in that** the para-phenylenediamines of formula (IV) are chosen from: para-phenylenediamine, para-tolylenediamine, 2-chloro-para-phenylenediamine, 2,3-dimethyl-para-phenylenediamine, 2,6-dimethyl-para-phenylenediamine, 2,6-diethyl-para-phenylenediamine, 2,5-dimethyl-para-phenylenediamine, N,N-dimethyl-para-phenylenediamine, N,N-diethyl-para-phenylenediamine, N,N-dipropyl-para-phenylenediamine, 4-amino-N,N-diethyl-3-methylaniline, N,N-bis(p-hydroxyethyl)-para-phenylenediamine, 4-N,N-bis(β-hydroxyethyl)amino-2-methylaniline, 4-N,N-bis(β-hydroxyethyl)amino-2-chloroaniline, 2-β-hydroxyethyl-para-phenylenediamine, 2-fluoro-para-phenylenediamine, 2-isopropyl-para-phenylenediamine, N-(β-hydroxypropyl)-para-phenylenediamine, 2-hydroxymethyl-para-phenylenediamine, N,N-dimethyl-3-methyl-para-phenylenediamine and N-ethyl-N-(β-hydroxyethyl)-para-phenylenediamine, and the addition salts thereof with an acid.

22. Composition according to Claim 19, **characterized in that** the double bases are chosen from the compounds of formula (V) below, and the addition salts thereof with an acid: 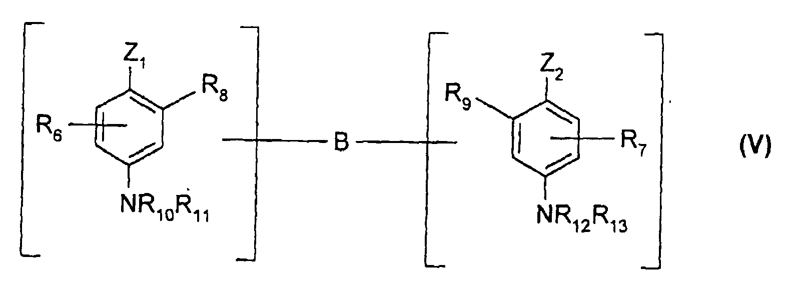 in which:
- Z₁ and Z₂, which may be identical or different, represent a hydroxyl or -NH₂ radical which can be substituted with a C₁-C₄ alkyl radical or with a linker arm B;
- the linker arm B represents a linear or branched alkylene chain containing from 1 to 14 carbon atoms, which can be interrupted or end with one or more nitrogenous groups and/or with one or more hetero atoms such as oxygen, sulphur or nitrogen atoms, and optionally substituted with one or more hydroxyl or C₁-C₆ alkoxy radicals;
- R₆ and R₇ represent a hydrogen or halogen atom, a C₁-C₄ alkyl radical, a C₁-C₄ monohydroxyalkyl radical, a C₂-C₄ polyhydroxyalkyl radical, a C₁-C₄ aminoalkyl radical or a linker arm B;
- R₈, R₉, R₁₀, R₁₁, R₁₂ and R₁₃, which may be identical or different, represent a hydrogen atom, a linker arm B or a C₁-C₄ alkyl radical;
it being understood that the compounds of formula (V) comprise only one linker arm B per molecule.

23. Composition according to Claim 20, **characterized in that** the double bases of formula (V) are chosen from N,N'-bis(β-hydroxyethyl)-N,N'-bis(4'-aminophenyl)-1,3-diaminopropanol, N,N'-bis(β-hydroxyethyl)-N,N'-bis(4'-aminophenyl)ethylenediamine, N,N'-bis(4-aminophenyl)tetramethylenediamine, N,N'-bis(β-hydroxyethyl)-N,N'-bis(4-aminophenyl)tetramethylenediamine, N,N'-bis(4-methylaminophenyl)tetramethylenediamine, N,N'-bis(ethyl)-N,N'-bis(4'-amino-3'-methylphenyl)-ethylenediamine and 1,8-bis(2,5-diaminophenoxy)-3,5-dioxaoctane, and the addition salts thereof with an acid.

24. Composition according to Claim 19, **characterized in that** the para-aminophenols are chosen from those of formula (VI) below, and the addition salts thereof with an acid: 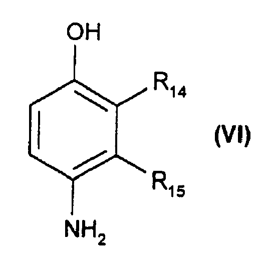 in which:
- R₁₄ and R₁₅, which may be identical or different, represent a hydrogen or halogen atom, a C₁-C₄ alkyl radical, a C₁-C₄ monohydroxyalkyl radical, a (C₁-C₉)alkoxy(C₁-C₄)alkyl radical, a C₁-C₄ aminoalkyl radical or a monohydroxy (C₁-C₄) alkylamino (C₁-C₉)alkyl radical,
it being understood that at least one of the radicals R₁₄ and R₁₅ represents a hydrogen atom.

25. Composition according to Claim 24, **characterized in that** the para-aminophenols of formula (VI) are chosen from para-aminophenol, 4-amino-3-methylphenol, 4-amino-3-fluorophenol, 4-amino-3-hydroxymethylphenol, 4-amino-2-methylphenol, 4-amino-2-hydroxymethylphenol, 4-amino-2-methoxymethylphenol, 4-amino-2-aminomethylphenol, 4-amino-2-(β-hydroxy-ethylaminomethyl)phenol and 4-amino-2-fluorophenol, and the addition salts thereof with an acid.

26. Composition according to Claim 19, **characterized in that** the heterocyclic bases are chosen from pyridine derivatives, pyrimidine derivatives including pyrazolopyrimidines, and pyrazole derivatives, and the addition salts thereof with an acid.

27. Composition according to Claim 19, **characterized in that** the couplers are meta-aminophenols, meta-phenylenediamines, meta-diphenols, naphthols or heterocyclic couplers, and the addition salts thereof with an acid.

28. Composition according to Claim 27, **characterized in that** the couplers are chosen from 2-methyl-5-aminophenol, 5-N-(β-hydroxyethyl)amino-2-methylphenol, 3-aminophenol, 1,3-dihydroxybenzene, 1,3-dihydroxy-2-methylbenzene, 4-chloro-1,3-dihydroxybenzene, 2,4-diamino-1-(β-hydroxyethyloxy)benzene, 2-amino-4-(β-hydroxyethylamino)-1-methoxybenzene, 1,3-diaminobenzene, 1,3-bis(2,4-diaminophenoxy)propane, sesamol, 1-amino-2-methoxy-4,5-methylenedioxybenzene, α-naphthol, 2-methyl-1-naphthol, 6-hydroxyindole, 4-hydroxyindole, 4-hydroxy-N-methylindole, 6-hydroxyindoline, 2,6-dihydroxy-4-methylpyridine, 1H-3-methylpyrazol-5-one and 1-phenyl-3-methylpyrazol-5-one, and the addition salts thereof with an acid.

29. Composition according to any one of Claims 18 to 28, **characterized in that** the oxidation dye(s) (bases and/or couplers) represent(s) from 0.001% to 20% by weight relative to the total weight of the ready-to-use dye composition.

30. Composition according to Claim 29, **characterized in that** the oxidation dye(s) represent(s) from 0.01% to 10% by weight relative to the total weight of the ready-to-use dye composition.

31. Composition according to any one of the preceding claims, **characterized in that** it contains one or more direct dyes.

32. Composition according to any one of the preceding claims, **characterized in that** it has a pH of between 3 and 11.

33. Composition according to any one of the preceding claims, **characterized in that** the medium which is suitable for dyeing is water or a mixture of water and at least one organic solvent.

34. Process for the oxidation dyeing of keratin fibres, and in particular human keratin fibres such as the hair, **characterized in that** a dye composition stored in the absence of air and comprising, in a medium which is suitable for dyeing, at least one oxidation dye (base and/or coupler) as defined in any one of Claims 18-30, and at least one enzymatic system comprising either (i) a 4-electron oxidoreductase, or (ii) a 2-electron oxidoreductase and at least one donor for the said oxidoreductase, or (iii) a peroxidase and optionally a donor for the said peroxidase, and a 2-electron oxidoreductase and a donor for the said oxidoreductase, is applied to the said fibres, in the presence of air, and at an application temperature of between room temperature and 60°C, for a period which is sufficient to develop the desired coloration, the enzymes being immobilized in a matrix of material obtained by acidic hydrolysis, or partial or total basic hydrolysis of organometallic precursors, followed by polycondensation, the organometallic precursors being metal oxides chosen from the group formed by:
(1) silanes, titanates or zirconates corresponding to one of the following formulae:
M(OR₁)₄ (Ia)
R-M(OR₁)₃ (Ib)
₃(OR₁)-M-(OR₁)₃ (Ic) 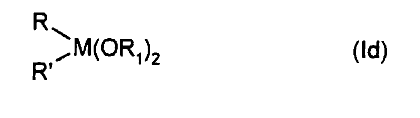
in which:
M denotes Si, Ti or Zr;
R₁ denotes a linear or branched, preferably C₁-C₄, alkyl radical;
R and R', independently of each other, denote a linear or branched C₁-C₃₀ alkyl radical, a C₁-C₃₀ cycloalkyl radical or an aryl or C₁-C₃₀ aralkyl or (C₁-C₃₀)-alkylaryl radical which may be substituted or unsubstituted, the said radicals R and R' possibly being substituted with one or more amino, carboxyl or hydroxyl groups, and mixtures thereof.

35. Process according to Claim 34, **characterized in that** the application temperature is between room temperature and 45°C.

36. Process according to Claim 34 or 35, **characterized in that** the period which is sufficient to develop the coloration is between 1 and 60 minutes.

37. Process according to Claim 36, **characterized in that** the period which is sufficient to develop the coloration is between 5 and 30 minutes.

38. Two-compartment dyeing device, **characterized in that** it comprises a first compartment containing a composition A comprising, in a medium which is suitable for dyeing, at least one oxidation dye (base and/or coupler) as defined in any one of Claims 18-30, and a second compartment containing a composition B comprising, in a medium which is suitable for dyeing, at least either (i) a 4-electron oxidoreductase, or (ii) a 2-electron oxidoreductase, or (iii) a peroxidase and a 2-electron oxidoreductase, composition A or composition B also containing at least one donor in the case of (ii) and (iii) in which the composition contains a 2-electron oxidoreductase, and compositions A and B possibly containing a donor for the peroxidase in the case of (iii), and the said enzymes being immobilized in a matrix of material obtained by partial or total acidic or basic hydrolysis of organometallic precursors, followed by polycondensation, the organometallic precursors being metal oxides chosen from the group formed by:
(1) silanes, titanates or zirconates corresponding to one of the following formulae:
M(OR₁)₄ (Ia)
R-M(OR₁)₃ (Ib)
₃(OR₁)-M-(OR₁)₃ (Ic) 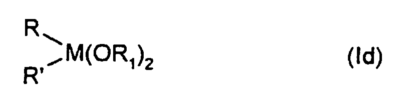
in which:
M denotes Si, Ti or Zr;
R₁ denotes a linear or branched, preferably C₁-C₄, alkyl radical;
R and R', independently of each other, denote a linear or branched C₁-C₃₀ alkyl radical, a C₁-C₃₀ cycloalkyl radical or an aryl or C₁-C₃₀ aralkyl or (C₁-C₃₀)-alkylaryl radical which may be substituted or unsubstituted, the said radicals R and R' possibly being substituted with one or more amino, carboxyl or hydroxyl groups, and mixtures thereof.

39. Three-compartment dyeing device comprising a first compartment containing a composition A comprising, in a medium which is suitable for dyeing, at least one oxidation dye (base and/or coupler) as defined in any one of Claims 18-30, a second compartment containing a composition B comprising, in a medium which is suitable for dyeing, at least one 2-electron oxidoreductase, a third compartment containing a composition C comprising, in a medium which is suitable for dyeing, at least one donor for the oxidoreductase, and the said enzymes being immobilized in a matrix of material obtained by acidic hydrolysis or partial or total basic hydrolysis of organometallic precursors, followed by polycondensation, the organometallic precursors being metal oxides chosen from the group formed by:
(1) silanes, titanates or zirconates corresponding to one of the following formulae:
M(OR₁)₄ (Ia)
R-M(OR₁)₃ (Ib)
₃(OR₁)-M-(OR₁)₃ (Ic) 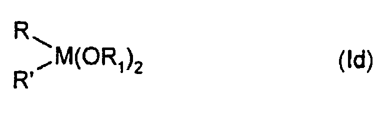
in which:
M denotes Si, Ti or Zr;
R₁ denotes a linear or branched, preferably C₁-C₄, alkyl radical;
R and R', independently of each other, denote a linear or branched C₁-C₃₀ alkyl radical, a C₁-C₃₀ cycloalkyl radical or an aryl or C₁-C₃₀ aralkyl or (C₁-C₃₀)-alkylaryl radical which may be substituted or unsubstituted, the said radicals R and R' possibly being substituted with one or more amino, carboxyl or hydroxyl groups, and mixtures thereof.

40. Three-compartment dyeing device comprising a first compartment containing a composition A comprising, in a medium which is suitable for dyeing, at least one oxidation dye (base and/or coupler) as defined in any one of Claims 18-30, a second compartment containing a composition B comprising, in a medium which is suitable for dyeing, at least one peroxidase and at least one 2-electron oxidoreductase, a third compartment containing a composition C comprising, in a medium which is suitable for dyeing, at least one donor for the oxidoreductase or a donor for the peroxidase,
composition A or composition B possibly containing one or more donors for the said enzymes,
and the said enzymes being immobilized in a matrix of material obtained by acidic hydrolysis, or partial or total basic hydrolysis of organometallic precursors, followed by polycondensation, the organometallic precursors being metal oxides chosen from the group formed by:
(1) silanes, titanates or zirconates corresponding to one of the following formulae:
M(OR₁)₄ (Ia)
R-M(OR₁)₃ (Ib)
₃(OR₁)-M-(OR₁)₃ (Ic) 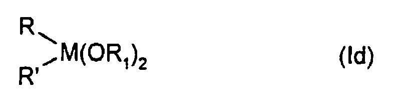
in which:
M denotes Si, Ti or Zr;
R₁ denotes a linear or branched, preferably C₁-C₄, alkyl radical;
R and R', independently of each other, denote a linear or branched C₁-C₃₀ alkyl radical, a C₁-C₃₀ cycloalkyl radical or an aryl or C₁-C₃₀ aralkyl or (C₁-C₃₀)-alkylaryl radical which may be substituted or unsubstituted, the said radicals R and R' possibly being substituted with one or more amino, carboxyl or hydroxyl groups.

41. Three-compartment dyeing device comprising a first compartment containing a composition A comprising, in a medium which is suitable for dyeing, at least one oxidation dye (base and/or coupler) as defined in any one of Claims 18-30, a second compartment containing a composition B comprising, in a medium which is suitable for dyeing, at least one peroxidase, a third compartment containing a composition C comprising, in a medium which is suitable for dyeing, at least one 2-electron oxidoreductase, or hydrogen peroxide,
composition A and/or composition B and/or composition C, indifferently, containing the donors for the said enzymes,
and the said enzymes being immobilized in a matrix of material obtained by acidic hydrolysis, or partial or total basic hydrolysis of organometallic precursors, followed by polycondensation, the organometallic precursors being metal oxides chosen from the group formed by:
(1) silanes, titanates or zirconates corresponding to one of the following formulae:
M(OR₁)₄ (Ia)
R-M(OR₁)₃ (Ib)
₃(OR₁)-M-(OR₁)₃ (Ic) 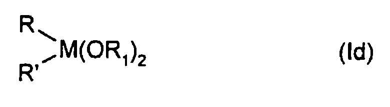
in which:
M denotes Si, Ti or Zr;
R₁ denotes a linear or branched, preferably C₁-C₄, alkyl radical;
R and R', independently of each other, denote a linear or branched C₁-C₃₀ alkyl radical, a C₁-C₃₀ cycloalkyl radical or an aryl or C₁-C₃₀ aralkyl or (C₁-C₃₀)-alkylaryl radical which may be substituted or unsubstituted, the said radicals R and R' possibly being substituted with one or more amino, carboxyl or hydroxyl groups, and mixtures thereof.

42. Process according to any one of Claims 34 to 37, **characterized in that** it comprises a preliminary step which consists in separately storing a composition A, a composition B and optionally a composition C as defined in any one of Claims 38-41, and then in mixing them together at the time of use in the presence of air, after which this mixture is applied to the keratin fibres.

## Patentansprüche

1. Gebrauchsfertige Zusammensetzung zum oxidativen Färben von Keratinfasern und insbesondere menschlichen Keratinfasern, wie Haaren, die in einem zum Färben geeigneten Medium mindestens einen Oxidationsfarbstoff und mindestens ein enzymatisches System enthält, das ausgewählt ist unter:
(a) mindestens einer 2-Elektronen-Oxidoreduktase in Kombination mit einem Donor,
(b) mindestens einer 4-Elektronen-Oxidoreduktase,
(c)
(i) mindestens einer Peroxidase gegebenenfalls in Kombination mit einem Donor und
(ii) Wasserstoffperoxid oder einem enzymatischen System, das Wasserstoffperoxid in situ bildet,
**dadurch gekennzeichnet, dass** das Enzym oder die Enzyme in einer Matrix aus einem Material immobilisiert sind, das durch saure Hydrolyse oder partielle oder vollständige basische Hydrolyse aus metallorganischen Vorläufern und anschließende Polykondensation erhalten wird, wobei die metallorganischen Vorläufer Metalloxide sind, die unter den folgenden Verbindungen und deren Gemischen ausgewählt sind:
(1) Silanen, Titanaten oder Zirconaten, die einer der folgenden Formeln entsprechen:
M(OR₁)₄ (Ia)
R-M(OR₁)₃ (Ib)
₃(OR₁)-M-(OR₁)₃ (Ic) 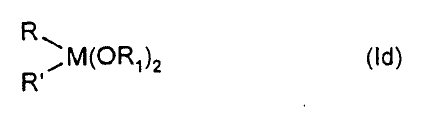
in den Formeln:
M bedeutet Si, Ti oder Zr;
R₁ bedeutet eine geradkettige oder verzweigte Alkylgruppe, die vorzugsweise 1 bis 4 Kohlenstoffatome aufweist;
R und R' bedeuten unabhängig voneinander eine geradkettige oder verzweigte C₁₋₃₀-Alkylgruppe, eine C₁₋₃₀-Cycloalkylgruppe, eine Aryl-, Aralkyl(C₁₋₃₀)- oder Alkyl(C₁₋₃₀)arylgruppe, die gegebenenfalls substituiert ist, wobei die Gruppen R und R' mit einer oder mehreren Aminogruppen, Carboxygruppen oder Hydroxygruppen substituiert sein können.

2. Zusammensetzung nach einem der Ansprüche 1 bis 3, **dadurch gekennzeichnet, dass** das Material bei Raumtemperatur durch saure oder basische Hydrolyse und Kondensation von Tetraalkoxysilan, Alkyltrialkoxysilan oder Aminoalkyltrialkoxysilan oder deren Gemischen über eine Sol-Gel-Reaktion hergestellt wird.

3. Zusammensetzung nach einem der Ansprüche 1 oder 2, **dadurch gekennzeichnet, dass** die 2-Elektronen-Oxidoreduktas(en) mit einem Donor für das Enzym oder die Enzyme verwendet werden und unter den Pyranose-Oxidasen, Glucose-Oxidasen, Glycerin-Oxidasen, Lactat-Oxidasen, Pyruvat-Oxidasen, Uricasen, Cholin-Oxidasen, Sarcosin-Oxidasen, Bilirubin-Oxidasen und Aminosäure-Oxidasen ausgewählt sind.

4. Zusammensetzung nach einem der Ansprüche 1 bis 3, **dadurch gekennzeichnet, dass** die 4-Elektronen-Oxidoreduktasen unter den Laccasen, Tyrosinasen, Catechol-Oxidasen und Polyphenol-Oxidasen ausgewählt sind.

5. Zusammensetzung nach Anspruch 4, **dadurch gekennzeichnet, dass** die 4-Elektronen-Oxidoreduktasen unter den Laccasen pflanzlicher Herkunft, Laccasen tierischer Herkunft, von Pilzen gebildeten Laccasen, Laccasen bakterieller Herkunft und den auf biotechnologischem Weg erhaltenen Laccasen ausgewählt sind.

6. Zusammensetzung nach Anspruch 5, **dadurch gekennzeichnet, dass** die Laccase pflanzlicher Herkunft ist und unter den Laccasen ausgewählt ist, die in Extrakten von Anacardiaceae, Podocarpaceae, Rosmarinus off., Solanum tuberosum, Iris sp., Coffea sp., Daucus carrota, Vinca minor, Persea americana, Catharenthus roseus, Musa sp., Malus pumila, Gingko biloba, Monotropa hypopithys (Fichtenspargel), Aesculus sp., Acer pseudoplatanus, Prunus persica und Pistacia palaestina enthalten sind.

7. Zusammensetzung nach Anspruch 5, **dadurch gekennzeichnet, dass** die Laccase von Pilzen stammt oder biotechnologisch hergestellt ist.

8. Zusammensetzung nach Anspruch 7, **dadurch gekennzeichnet, dass** die Laccase unter den Laccasen ausgewählt ist, die von Polyporus versicolor, Rhizoctonia praticola, Rhus vernicifera, Scytalidium, Polyporus pinsitus, Myceliophtora thermophila, Rhizoctonia solani, Pyricularia oryzae, Trametes versicolor, Fomes fomentarius, Chaetomium thermophile, Neurospora crassa, Colorius versicol, Botrytis cinerea, Rigidoporus lignosus, Phellinus noxius, Pleurotus ostreatus, Aspergillus nidulans, Podospora anserina, Agaricus bisporus, Ganoderma lucidum, Glomerella cingulata, Lactarius piperatus, Russula delica, Heterobasidion annosum, Thelephora terrestris, Cladosporium cladosporioides, Cerrena unicolor, Coriolus hirsutus, Ceriporiopsis subvermispora, Coprinus cinereus, Panaeolus papilionaceus, Panaeolus sphinctrinus, Schizophyllum commune, Dichomitus squalens und deren Varianten stammen.

9. Zusammensetzung nach einem der vorhergehenden Ansprüche, **dadurch gekennzeichnet, dass** die Oxidoreduktasen, die 2 oder 4 Elektronen übertragen, 0,01 bis 20 Gew.-% des Gesamtgewichts der gebrauchsfertigen Farbmittelzusammensetzung ausmachen.

10. Zusammensetzung nach Anspruch 9, **dadurch gekennzeichnet, dass** die Oxidoreduktasen, die 2 oder 4 Elektronen übertragen, 0,1 bis 10 Gew.-% des Gesamtgewichts der gebrauchsfertigen Farbmittelzusammensetzung ausmachen.

11. Zusammensetzung nach einem der Ansprüche 1 oder 2, **dadurch gekennzeichnet, dass** die Peroxidase(n) einfache Peroxidasen sind.

12. Zusammensetzung nach einem der Ansprüche 1 oder 2, **dadurch gekennzeichnet, dass** die Peroxidase(n) Katalasen sind.

13. Zusammensetzung nach einem der Ansprüche 1 oder 2, **dadurch gekennzeichnet, dass** die Peroxidase(n) mit einem Donor für das Enzym oder die Enzyme verwendet werden und unter den NADH-Peroxidasen, Fettsäure-Peroxidasen, NADPH-Peroxidasen, Cytochrom-c-Peroxidasen, Iodid-Peroxidasen, Chlorid-Peroxidasen, L. Ascorbaten und Glutathion-Peroxidasen ausgewählt sind.

14. Zusammensetzung nach einem der Ansprüche 1 oder 2, **dadurch gekennzeichnet, dass** die Peroxidase(n) tierischer Herkunft, pflanzlicher Herkunft, von Pilzen gebildet, bakterieller Herkunft oder auf biotechnologischem Weg erhalten sind.

15. Zusammensetzung nach Anspruch 14, **dadurch gekennzeichnet, dass** die Peroxidase(n) aus Apfel, Aprikose, Gerste, Schwarzem Rettich, Rübe, Kohl, Karotte, Mais, Baumwolle, Lauch, Traube, Minze, Rhabarber, Soja, Spinat, Coprinus, Kuhmilch, Mikroorganismen vom Typ Acetobacter peroxidans, Staphylococcus faecalis, Arthromyces ramosus stammen.

16. Zusammensetzung nach einem der Ansprüche 11 bis 15, **dadurch gekennzeichnet, dass** die Peroxidase(n) 0,0001 bis 20 Gew.-% des Gesamtgewichts der gebrauchsfertigen Farbmittelzusammensetzung ausmachen.

17. Zusammensetzung nach Anspruch 16, **dadurch gekennzeichnet, dass** die Peroxidase(n) 0,001 bis 10 Gew.-% des Gesamtgewichts der gebrauchsfertigen Farbmittelzusammensetzung ausmachen.

18. Zusammensetzung nach einem der vorhergehenden Ansprüche, **dadurch gekennzeichnet, dass** der oder die Oxidationsfarbstoff(e) unter den Oxidationsbasen und/oder Kupplern ausgewählt sind.

19. Zusammensetzung nach Anspruch 18, **dadurch gekennzeichnet, dass** die Oxidationsbasen unter den p-Phenylendiaminen, Doppelbasen, p-Aminophenolen und den heterocyclischen Basen und ihren Additionssalzen mit einer Säure ausgewählt sind.

20. Zusammensetzung nach Anspruch 19, **dadurch gekennzeichnet, dass** die p-Phenylendiamine unter den Verbindungen der folgenden Formel (IV) und deren Additionssalzen mit einer Säure ausgewählt sind: 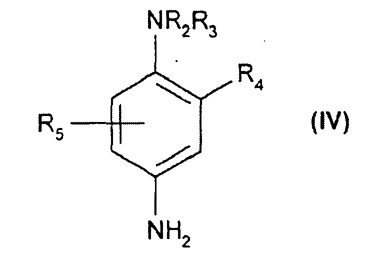 worin bedeuten:
- R₂ und R₃, die gleich oder verschieden sind, ein Wasserstoffatom, eine C₁₋₄-Alkylgruppe oder eine C₁₋₄-Monohydroxyalkylgruppe,
- R₄ ein Wasserstoff- oder Halogenatom, eine C₁₋₄-Alkylgruppe oder eine C₁₋₄-Monohydroxyalkylgruppe,
- R₅ ein Wasserstoffatom oder eine C₁₋₄-Alkylgruppe.

21. Zusammensetzung nach Anspruch 20, **dadurch gekennzeichnet, dass** die p-Phenylendiamine der Formel (IV) ausgewählt sind unter:
p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-N,N-diethyl-3-methyl-anilin, N,N-Bis(β-hydroxyethyl)-p-phenylendiamin, 4-N,N-Bis(β-hydroxyethyl)amino-2-methylanilin, 4-N,N-Bis(β-hydroxyethyl)amino-2-chlor-anilin, 2-β-Hydroxyethyl-p-phenylendiamin, 2-Fluor-p-phenylendiamin, 2-Isopropyl-p-phenylendiamin, N-(β-Hydroxypropyl)-p-phenylendiamin, 2-Hydroxymethyl-p-phenylendiamin, N,N-Dimethyl-3-methyl-p-phenylendiamin, N,N-(Ethyl, β-hydroxyethyl)-p-phenylendiamin und deren Additionssalzen mit einer Säure.

22. Zusammensetzung nach Anspruch 19, **dadurch gekennzeichnet, dass** die Doppelbasen unter den Verbindungen der folgenden Formel (V) und deren Additionssalze mit einer Säure ausgewählt sind: 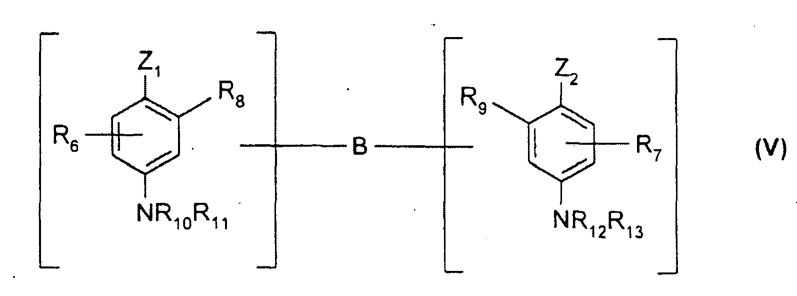 worin bedeuten:
- Z₁ und Z₂, die identisch oder voneinander verschieden sind, , eine Hydroxygruppe oder eine NH₂-Gruppe, die mit einer C₁₋₄-Alkylgruppe oder einer Verbindungsgruppe B substituiert sein können,
- die Verbindungsgruppe B eine geradkettige oder verzweigte Alkylengruppe mit 1 bis 14 Kohlenstoffatomen, die durch eine oder mehrere stickstoffhaltige Gruppen und/oder ein oder mehrere Heteroatome, wie Sauerstoff, Schwefel oder Stickstoff, unterbrochen oder abgeschlossen werden kann und gegebenenfalls mit einer oder mehreren Hydroxy- oder C₁₋₆-Alkoxygruppen substituiert sein kann,
- R₆ und R₇ ein Wasserstoff- oder Halogenatom, eine C₁₋₄-Alkylgruppe, eine C₁₋₄-Monohydroxyalkylgruppe, eine C₂₋₄-Polyhydroxyalkylgruppe, eine C₁₋₄-Aminoalkylgruppe oder eine Verbindungsgruppe B,
- die Gruppen R₈, R₉, R₁₀, R₁₁, R₁₂ und R₁₃, die identisch oder voneinander verschieden sind, ein Wasserstoffatom, eine Verbindungsgruppe B oder eine C₁₋₄-Alkylgruppe, mit der Maßgabe, dass die Verbindungen der Formel (V) nur eine Verbindungsgruppe B pro Molekül aufweisen.

23. Zusammensetzung nach Anspruch 20, **dadurch gekennzeichnet, dass** die Doppelbasen der Formel (V) unter N,N'-Bis(β-hydroxyethyl)-N,N'-bis-(4'-aminophenyl)-1,3-diaminopropanol, N,N'-Bis(β-hydroxyethyl)-N,N'-bis(4'-aminophenyl)-ethylendiamin, N,N'-Bis(4-aminophenyl)-tetramethylendiamin, N,N'-Bis(β-hydroxyethyl)-N,N'-bis(4-aminophenyl)-tetramethylendiamin, N,N'-Bis(4-methylaminophenyl)-tetramethylendiamin, N,N'-Bis(ethyl)-N,N'-bis-(4'-amino, 3'-methylphenyl)-ethylendiamin, 1,8-Bis(2,5-diaminophenoxy)-3,5-dioxaoctan und deren Additionssalzen mit einer Säure ausgewählt sind.

24. Zusammensetzung nach Anspruch 19, **dadurch gekennzeichnet, dass** die p-Aminopheriole unter den Verbindungen der folgenden Formel (VI) und deren Additionssalze mit einer Säure ausgewählt sind: 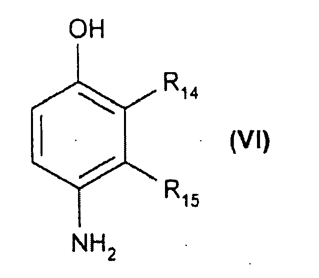 worin bedeuten:
- R₁₄ und R₁₅, die gleich oder verschieden sind, ein Wasserstoff- oder Halogenatom, eine C₁₋₄-Alkylgruppe, eine C₁₋₄-Mono-hydroxyalkylgruppe, eine C₁₋₄-Alkoxy-C₁₋₄-alkylgruppe, eine C₁₋₄-Aminoalkylgruppe oder eine C₁₋₄-Monohydroxyalkyl-C₁₋₄-aminoalkylgruppe,
mit der Maßgabe, dass mindestens eine der Gruppen R₁₄ oder R₁₅ ein Wasserstoffatom bedeutet.

25. Zusammensetzung nach Anspruch 24, **dadurch gekennzeichnet, dass** die p-Aminophenole der Formel (VI) unter p-Aminophenol, 4-Amino-3-methyl-phenol, 4-Amino-3-fluor-phenol, 4-Amino-3-hydroxymethyl-phenol, 4-Amino-2-methyl-phenol, 4-Amino-2-hydroxymethyl-phenol, 4-Amino-2-methoxymethyl-phenol, 4-Amino-2-aminomethyl-phenol, 4-Amino-2-(β-hydroxyethylaminomethyl)-phenol, 4-Amino-2-fluor-phenol und deren Additionssalzen mit einer Säure ausgewählt sind.

26. Zusammensetzung nach Anspruch 19, **dadurch gekennzeichnet, dass** die heterocyclischen Basen unter den Pyridinderivaten, Pyrimidinderivaten und darunter Pyrazolopyrimidinen, Pyrazolderivaten und deren Additionssalzen mit einer Säure ausgewählt sind.

27. Zusammensetzung nach Anspruch 19, **dadurch gekennzeichnet, dass** die Kuppler unter den m-Aminophenolen, m-Phenylendiaminen, m-Dihydroxybenzolen, Naphtholen oder den heterocyclischen Kupplern und deren Additionssalzen mit einer Säure ausgewählt sind.

28. Zusammensetzung nach Anspruch 27, **dadurch gekennzeichnet, dass** die Kuppler unter 2-Methyl-5-amino-phenol, 5-N-(β-Hydroxyethyl)amino-2-methyl-phenol, 3-Amino-phenol, 1,3-Dihydroxy-benzol, 1,3-Dihydroxy-2-methyl-benzol, 4-Chlor-1,3-dihydroxy-benzol, 2,4-Diamino-1-(β-hydroxyethyloxy)-benzol, 2-Amino-4-(β-hydroxyethylamino)-1-methoxy-benzol, 1,3-Diaminobenzol, 1,3-Bis(2,4-diaminophenoxy)-propan, Sesamol, 1-Amino-2-methoxy-4,5-methylendioxy-benzol, α-Naphthol, 2-Methyl-1-naphthol, 6-Hydroxyindol, 4-Hydroxyindol, 4-Hydroxy-N-methylindol, 6-Hydroxyindolin, 2,6-Dihydroxy-4-methyl-pyridin, 1-H-3-Methyl-pyrazol-5-on, 1-Phenyl-3-methyl-pyrazol-5-on und den Additionssalzen dieser Verbindungen mit einer Säure ausgewählt sind.

29. Zusammensetzung nach einem der Ansprüche 18 bis 28, **dadurch gekennzeichnet, dass** der oder die Oxidationsfarbstoff(e) (Basen und/oder Kuppler) 0,001 bis 20 Gew.-% des Gesamtgewichts der gebrauchsfertigen Farbmittelzusammensetzung ausmachen.

30. Zusammensetzung nach Anspruch 29, **dadurch gekennzeichnet, dass** der oder die Oxidationsfarbstoff(e) 0,01 bis 10 Gew.-% des Gesamtgewichts der gebrauchsfertigen Farbmittelzusammensetzung ausmachen.

31. Zusammensetzung nach einem der vorhergehenden Ansprüche, **dadurch gekennzeichnet, dass** sie einen oder mehrere Direktfarbstoffe enthält.

32. Zusammensetzung nach einem der vorhergehenden Ansprüche, **dadurch gekennzeichnet, dass** sie einen pH-Wert im Bereich von 3 bis 11 aufweist.

33. Zusammensetzung nach einem der vorhergehenden Ansprüche, **dadurch gekennzeichnet, dass** das zum Färben geeignete Medium aus Wasser oder einem Gemisch von Wasser und mindestens einem organischen Lösungsmittel besteht.

34. Verfahren zum oxidativen Färben von Keratinfasern und insbesondere menschlichen Keratinfasern, wie zum Färben der Haare, **dadurch gekennzeichnet, dass** auf die Fasern bei einer Applikationstemperatur im Bereich von Raumtemperatur bis 60 °C und während einer Zeitspanne, die für die Bildung der gewünschten Färbung ausreichend ist, in Gegenwart von Luft eine unter Luftabschluss aufbewahrte Farbmittelzusammensetzung aufgebracht wird, die in einem zum Färben geeigneten Medium mindestens einen Oxidationsfarbstoff (Base und/oder Kuppler), wie er in einem der Ansprüche 18 bis 30 definiert ist, und mindestens ein enzymatisches System enthält, das entweder (i) eine 4-Elektronen-Oxidoreduktase oder (ii) eine 2-Elektronen-Oxidoreduktase und mindestens einen Donor für die Oxidoreduktase oder (iii) eine Peroxidase und gegebenenfalls einen Donor für die Peroxidase und eine 2-Elektronen-Oxidoreduktase und einen Donor für die Oxidoreduktase enthält, wobei die Enzyme in einer Matrix aus einem Material immobilisiert sind, das durch saure Hydrolyse oder partielle oder vollständige basische Hydrolyse aus metallorganischen Vorläufern und anschließende Polykondensation erhalten wird, wobei die metallorganischen Vorläufer Metalloxide sind, die unter den (1) Silanen, Titanaten oder Zirconaten, die einer der folgenden Formeln entsprechen:
N(OR₁)₍₄₋ₜ₎(X)ₜ (IIIa)
R-N(OR₁)₃₋ₜ(X)ₜ (IIIb)
ₜ(X)₃₋ₜ(OR₁)N-R-N(OR₁)₃₋ₜ(X)ₜ (IIIc) 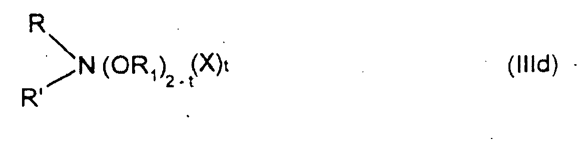
in den Formeln:
M bedeutet Si, Ti oder Zr;
R₁ bedeutet eine geradkettige oder verzweigte Alkylgruppe, die vorzugsweise 1 bis 4 Kohlenstoffatome aufweist;
R und R' bedeuten unabhängig voneinander eine geradkettige oder verzweigte C₁₋₃₀-Alkylgruppe, eine C₁₋₃₀-Cycloalkylgruppe, eine Aryl-, Aralkyl(C₁₋₃₀)- oder Alkyl(C₁₋₃₀)arylgruppe, die gegebenenfalls substituiert ist, wobei die Gruppen R und R' mit einer oder mehreren Aminogruppen, Carboxylgruppen oder Hydroxygruppen substituiert sein können,
und deren Gemischen ausgewählt sind.

35. Verfahren nach Anspruch 34, **dadurch gekennzeichnet, dass** die Applikationstemperatur im Bereich von Raumtemperatur bis 45 °C liegt.

36. Verfahren nach Anspruch 34 oder 35, **dadurch gekennzeichnet, dass** die Zeitspanne, die zur Entwicklung der gewünschten Färbung ausreichend ist, im Bereich von 1 bis 60 min liegt.

37. Verfahren nach Anspruch 36, **dadurch gekennzeichnet, dass** die Zeitspanne, die zur Entwicklung der gewünschten Färbung ausreichend ist, im Bereich von 5 bis 30 min liegt.

38. Vorrichtung zum Färben mit zwei Abteilungen, **dadurch gekennzeichnet, dass** sie eine erste Abteilung mit einer Zusammensetzung A, die in einem zum Färben geeigneten Medium mindestens einen Oxidationsfarbstoff (Base und/oder Kuppler), wie er in einem der Ansprüche 18 bis 30 definiert ist, und eine zweite Abteilung mit einer Zusammensetzung B umfasst, die in einem zum Färben geeigneten Medium zumindest entweder (i) eine 4-Elektronen-Oxidoreduktase oder (ii) eine 2-Elektronen-Oxidoreduktase oder (iii) eine Peroxidase und eine 2-Elektronen-Oxidoreduktase enthält, wobei die Zusammensetzung A oder die Zusammensetzung B für die Fälle (ii) und (iii) ferner mindestens einen Donor enthält, wenn die Zusammensetzung eine 2-Elektronen-Oxidoreduktase enthält, und die Zusammensetzungen A und B für den Fall (iii) einen Donor für die Peroxidase enthalten können und wobei die Enzyme in einer Matrix aus einem Material immobilisiert sind, das durch saure Hydrolyse oder partielle oder vollständige basische Hydrolyse aus metallorganischen Vorläufern und anschließende Polykondensation erhalten wird, wobei die metallorganischen Vorläufer Metalloxide sind, die unter den (1) Silanen, Titanaten oder Zirconaten, die einer der folgenden Formeln entsprechen:
N (OR₁)₍₄₋ₜ₎(X)ₜ (IIIa)
R-N(OR₁)₃₋ₜ(X)ₜ (IIIb)
ₜ(X)₃₋ₜ(OR₁)N-R-N(OR₁)₃₋ₜ(X)ₜ (IIIc) 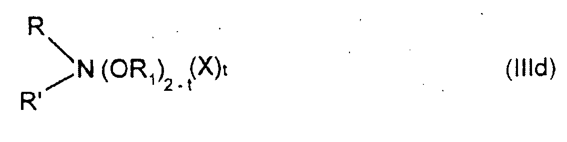
in den Formeln:
M bedeutet Si, Ti oder Zr;
R₁ bedeutet eine geradkettige oder verzweigte Alkylgruppe, die vorzugsweise 1 bis 4 Kohlenstoffatome aufweist;
R und R' bedeuten unabhängig voneinander eine geradkettige oder verzweigte C₁₋₃₀-Alkylgruppe, eine C₁₋₃₀-Cycloalkylgruppe, eine Aryl-, Aralkyl(C₁₋₃₀)- oder Alkyl(C₁₋₃₀)arylgruppe, die gegebenenfalls substituiert ist, wobei die Gruppen R und R' mit einer oder mehreren Aminogruppen, Carboxygruppen oder Hydroxygruppen substituiert sein können,
und deren Gemischen ausgewählt sind.

39. Vorrichtung zum Färben mit drei Abteilungen, die eine erste Abteilung mit einer Zusammensetzung A, die in einem zum Färben geeigneten Medium mindestens einen Oxidationsfarbstoff (Base und/oder Kuppler), wie er in einem der Ansprüche 18 bis 30 definiert ist, eine zweite Abteilung mit einer Zusammensetzung B, die in einem zum Färben geeigneten Medium mindestens eine 2-Elektronen-Oxidoreduktase enthält, und eine dritte Abteilung mit einer Zusammensetzung C umfasst, die in einem zum Färben geeigneten Medium mindestens einen Donor für die Oxidoreduktase enthält, wobei die Enzyme in einer Matrix aus einem Material immobilisiert sind, das durch saure Hydrolyse oder partielle oder vollständige basische Hydrolyse aus metallorganischen Vorläufern und anschließende Polykondensation erhalten wird, wobei die metallorganischen Vorläufer Metalloxide sind, die unter den (1) Silanen, Titanaten oder Zirconaten, die einer der folgenden Formeln entsprechen:
N (OR₁)₍₄₋ₜ₎(X)ₜ (IIIa)
R-N(OR₁)₃₋ₜ(X)ₜ (IIIb)
ₜ(X)₃₋ₜ(OR₁)N-R-N(OR₁)₃₋ₜ(X)ₜ (IIIc) 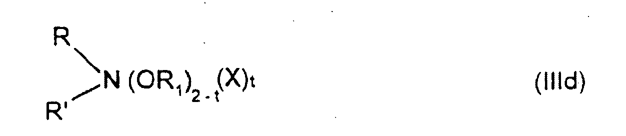
in den Formeln:
M bedeutet Si, Ti oder Zr;
R₁ bedeutet eine geradkettige oder verzweigte Alkylgruppe, die vorzugsweise 1 bis 4 Kohlenstoffatome aufweist;
R und R' bedeuten unabhängig voneinander eine geradkettige oder verzweigte C₁₋₃₀-Alkylgruppe, eine C₁₋₃₀-Cycloalkylgruppe, eine Aryl-, Aralkyl(C₁₋₃₀)- oder Alkyl(C₁₋₃₀)arylgruppe, die gegebenenfalls substituiert ist, wobei die Gruppen R und R' mit einer oder mehreren Aminogruppen, Carboxygruppen oder Hydroxygruppen substituiert sein können,
und deren Gemischen ausgewählt sind.

40. Vorrichtung zum Färben mit drei Abteilungen, die eine erste Abteilung mit einer Zusammensetzung A, die in einem zum Färben geeigneten Medium mindestens einen Oxidationsfarbstoff (Base und/oder Kuppler), wie er in einem der Ansprüche 18 bis 30 definiert ist, eine zweite Abteilung mit einer Zusammensetzung B, die in einem zum Färben geeigneten Medium mindestens eine Peroxidase und mindestens eine 2-Elektronen-Oxidoreduktase enthält, und eine dritte Abteilung mit einer Zusammensetzung C umfasst, die in einem zum Färben geeigneten Medium mindestens einen Donor für die Oxidoreduktase oder einen Donor für die Peroxidase enthält,
wobei die Zusammensetzung A oder die Zusammensetzung B einen oder mehrere Donoren für die Enzyme enthalten können und
wobei die Enzyme in einer Matrix aus einem Material immobilisiert sind, das durch saure Hydrolyse oder partielle oder vollständige basische Hydrolyse aus metallorganischen Vorläufern und anschließende Polykondensation erhalten wird,
wobei die metallorganischen Vorläufer Metalloxide sind, die unter den (1) Silanen, Titanaten oder Zirconaten, die einer der folgenden Formeln entsprechen:
N(OR₁)₍₄₋ₜ₎(X)ₜ (IIIa)
R-N(OR₁)₃₋ₜ(X)ₜ (IIIb)
ₜ(X)₃₋ₜ(OR₁)N-R-N(OR₁)₃₋ₜ(X)ₜ (IIIc) 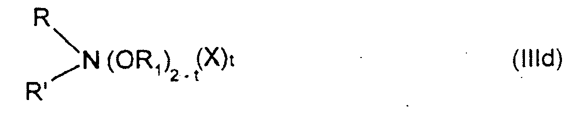
in den Formeln:
M bedeutet Si, Ti oder Zr;
R₁ bedeutet eine geradkettige oder verzweigte Alkylgruppe, die vorzugsweise 1 bis 4 Kohlenstoffatome aufweist;
R und R' bedeuten unabhängig voneinander eine geradkettige oder verzweigte C₁₋₃₀-Alkylgruppe, eine C₁₋₃₀-Cycloalkylgruppe, eine Aryl-, Aralkyl(C₁₋₃₀)- oder Alkyl(C₁₋₃₀)arylgruppe, die gegebenenfalls substituiert ist, wobei die Gruppen R und R' mit einer oder mehreren Aminogruppen, Carboxygruppen oder Hydroxygruppen substituiert sein können,
und deren Gemischen ausgewählt sind.

41. Vorrichtung zum Färben mit drei Abteilungen, die eine erste Abteilung mit einer Zusammensetzung A, die in einem zum Färben geeigneten Medium mindestens einen Oxidationsfarbstoff (Base und/oder Kuppler), wie er in einem der Ansprüche 18 bis 30 definiert ist, eine zweite Abteilung mit einer Zusammensetzung B, die in einem zum Färben geeigneten Medium mindestens eine Peroxidase enthält, und eine dritte Abteilung mit einer Zusammensetzung C umfasst, die in einem zum Färben geeigneten Medium mindestens eine 2-Elektronen-Oxidoreduktase oder Wasserstoffperoxid enthält,
wobei die Zusammensetzung A und/oder die Zusammensetzung B und/oder die Zusammensetzung C Donoren für die Enzyme enthalten und
wobei die Enzyme in einer Matrix aus einem Material immobilisiert sind, das durch saure Hydrolyse oder partielle oder vollständige basische Hydrolyse aus metallorganischen Vorläufern und anschließende Polykondensation erhalten wird,
wobei die metallorganischen Vorläufer Metalloxide sind, die unter den (1) Silanen, Titanaten oder Zirconaten, die einer der folgenden Formeln entsprechen:
N(OR₁)₍₄₋ₜ₎(X)ₜ (IIIa)
R-N(OR₁)₃₋ₜ(X)ₜ (IIIb)
ₜ(X)₃₋ₜ(OR₁)N-R-N(OR₁)₃₋ₜ(X)ₜ (IIIc) 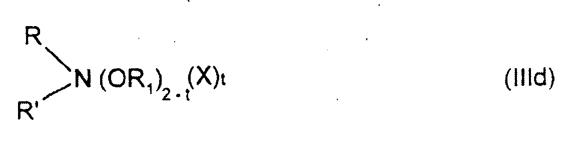
in den Formeln:
M bedeutet Si, Ti oder Zr;
R₁ bedeutet eine geradkettige oder verzweigte Alkylgruppe, die vorzugsweise 1 bis 4 Kohlenstoffatome aufweist;
R und R' bedeuten unabhängig voneinander eine geradkettige oder verzweigte C₁₋₃₀-Alkylgruppe, eine C₁₋₃₀-Cycloalkylgruppe, eine Aryl-, Aralkyl(C₁₋₃₀)- oder Alkyl(C₁₋₃₀)arylgruppe, die gegebenenfalls substituiert ist, wobei die Gruppen R und R' mit einer oder mehreren Aminogruppen, Carboxygruppen oder Hydroxygruppen substituiert sein können,
und deren Gemischen ausgewählt sind.

42. Verfahren nach einem der Ansprüche 34 bis 37, **dadurch gekennzeichnet, dass** es einen vorbereitenden Schritt umfasst, der darin besteht, eine Zusammensetzung A, eine Zusammensetzung B und gegebenenfalls eine Zusammensetzung C, wie sie in einem der Ansprüche 38 bis 41 definiert sind, getrennt voneinander aufzubewahren und bei der Anwendung in Gegenwart von Luft zu vermischen, bevor das Gemisch auf die Keratinfasern aufgetragen wird.
